(19) Europäisches Patentamt
European Patent Office
Office européen des brevets

(11) Veröffentlichungsnummer: **0 228 018 B1**

(12) # EUROPÄISCHE PATENTSCHRIFT

(45) Veröffentlichungstag der Patentschrift: **02.01.92**

(51) Int. Cl.[5]: **C12N 15/27**, C12P 21/02, C12N 1/20, A61K 37/02, //(C12N1/20,C12R1:19)

(21) Anmeldenummer: **86117484.5**

(22) Anmeldetag: **16.12.86**

(54) **GM-CSF-Protein, seine Derivate, Herstellung solcher Proteine und ihre Verwendung.**

(30) Priorität: **21.12.85 DE 3545568**

(43) Veröffentlichungstag der Anmeldung:
**08.07.87 Patentblatt 87/28**

(45) Bekanntmachung des Hinweises auf die Patenterteilung:
**02.01.92 Patentblatt 92/01**

(84) Benannte Vertragsstaaten:
**AT BE CH DE ES FR GB GR IT LI LU NL SE**

(56) Entgegenhaltungen:
**EP-A- 0 128 733**
**EP-A- 0 183 350**
**WO-A-86/00639**
**WO-A-86/03225**
**US-A- 4 438 032**

**PROCEEDINGS OF THE NATIONAL ACADEMY OF SCIENCES OF THE USA, Band 82, Nr. 18, September 1985, Seiten 6250-6254, Washington, US; M.A. CANTRELL et al.: "Cloning, sequence, and expression of a human granulocyte/macrophage colony-stimulating factor"**

(73) Patentinhaber: **HOECHST AKTIENGESELLSCHAFT**
**Postfach 80 03 20**
**W-6230 Frankfurt am Main 80(DE)**

(72) Erfinder: **Habermann, Paul, Dr.**
**Heimchenweg 80**
**W-6230 Frankfurt am Main 80(DE)**
Erfinder: **Müllner, Hubert, Dr.**
**Berliner Ring 20**
**W-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Humaner Granulozyten-Makrophagen-"Colony-Stimulating"-Faktor (GM-CSF) ist ein Glykoprotein mit einem Molgewicht von etwa 23 000 Dalton. Die cDNA-Sequenz und die Expression des Glykoproteins in Säugerzellen sind bereits bekannt (G.G. Wong et al., Science 228 (1985), 810-815, D. Metcalf, Science 229 (1985), 16-22).

Die Erfindung betrifft humane Granulozyten-Makrophagen-"Colony-Stimulating"-Faktor-Proteine (GM-CSF) der Formel

Pro-$(As)_x$-CSF(12-126)-Z,

in der $(As)_x$ ganz oder teilweise die ersten 11 Aminosäuren der natürlichen GM-CSF-Sequenz und Z Glu oder Asp bedeuten, im folgenden "CSF". Weitere Aspekte der Erfindung und ihre bevorzugten Ausgestaltungen sind im folgenden näher erläutert bzw. in den Patentansprüchen definiert.

Die Erfindung betrifft weiterhin die Herstellung von CSF durch Expression in Bakterien, insbesondere in E. coli. Hierfür können die publizierten cDNA-Sequenzen herangezogen werden, die in an sich bekannter Weise, vorzugsweise synthetisch, gewonnen werden können.

Die Erfindung betrifft außerdem Expressionsvektoren zur Anwendung in Bakterien, insbesondere in E. coli, die eine für CSF bzw. ein CSF-Fusionsprotein codierende DNA in geeigneter Anordnung ("operatively linked to") enthalten.

Die Erfindung betrifft außerdem biologisch aktive Derivate des CSF, die durch an sich bekannte Abwandlungen der DNA-Sequenzen zugänglich sind. So können beispielsweise bei der Konstruktion von Vektoren für Fusionsproteine Spaltstellen eingebaut werden, die nach Abspaltung des CSF-Proteins C-terminale und/oder N-terminale Abwandlungen in der Aminosäuresequenz aufweisen. Darüber hinaus betrifft die Erfindung die Anwendung solcher Proteine in Heilverfahren bzw. ihre Verwendung zur Herstellung von Arzneimitteln sowie Arzneimittel, die CSF-Protein und seine biologisch aktiven Derivate enthalten, insbesondere Arzneimittel zur Stimulation der Proliferation haematopoetischer Zellen und zur Förderung der Granulozyten- und Makrophagen-Bildung.

Zur Erläuterung der Erfindung dienen weiterhin die Figuren 1 bis 15, die jeweils, meist in Form eines Fließschemas, die Verfahren der Beispiele gleicher Nummer veranschaulichen. Diese Figuren sind nicht maßstäblich, insbesondere im Bereich der Polylinker ist der Maßstab "gedehnt".

So zeigen die Figur 1 und ihre Fortsetzungen 1a und 1b die Herstellung des Vektors pW 225, der zur Direktexpression von (Met-)CSF dient.

Dieses Produkt kann durch Säurespaltung in erfindungsgemäße CSF überführt werden. Die folgenden Figuren beziehen sich auf Vektoren, die zur Expression von Fusionsproteinen führen, in welchen N-terminal vor der CSF-Aminosäuresequenz ein "Ballast"-Protein angeordnet ist, das sich vor einer Teilsequenz des humanen Interleukin-2, im folgenden "IL-2" bzw. "ΔIL-2", ableitet:

Figur 2 und ihre Fortsetzungen 2a und 2b zeigen die Herstellung des Vektors pW 216, der für ein Fusionsprotein codiert, aus welchem durch Säurespaltung ein CSF-Derivat hervorgeht, das N-terminal um die Aminosäure Prolin verlängert ist.

Die Figur 3 zeigt die Synthese des Vektors pW 240, der für ein Fusionsprotein codiert, das nach Säurespaltung zu einem CSF-Derivat führt, welches an Stelle der ersten Aminosäure (Alanin) Prolin trägt.

Die Figur 4 bezieht sich auf die Herstellung des Vektors pW 241, der für ein Fusionsprotein codiert, welches nach Säurespaltung zu einem CSF-Derivat führt, bei dem die erste Aminosäure (Alanin) fehlt.

Die Figur 5 demonstriert die Herstellung des Vektors pW 242, der für ein Fusionsprotein codiert, das nach Säurespaltung zu einem CSF-Derivat führt, bei dem die ersten 5 Aminosäuren eliminiert sind.

Die Figur 6 bezieht sich auf die Herstellung des Vektors pW 243, der für ein Fusionsprotein codiert, welches nach Säurespaltung zu eine CSF-Derivat führt, bei dem die ersten 7 Aminosäuren fehlen.

Die Figur 7 zeigt die Synthese des Vektors pW 244, der für ein Fusionsprotein codiert, bei dem nach Säurespaltung ein CSF-Derivat erhalten wird, bei dem die ersten 11 Aminosäuren eliminiert sind.

Die Figur 8 und ihre Fortsetzung 8a zeigen die Synthese des Vektors pW 246. Dieser codiert für ein Fusionsprotein, bei dem auf die IL-2-Teilsequenz zwei variierte, als "CSF′" bezeichnete Sequenzen folgen. Nach Säurespaltung erhält man ein CSF-Derivat, bei dem N-terminal vor der ersten Aminosäure Prolin steht und bei dem die letzte Aminosäure durch Asparaginsäure ersetzt ist.

Die Figur 9 zeigt die Synthese des Vektors pW 247, der für ein Fusionsprotein codiert, bei dem auf die IL-2-Teilsequenz drei CSF'-Sequenzen folgen. Nach Säurespaltung wird das bei Figur 8 charakterisierte CSF-Derivat erhalten.

Die Figur 10 und ihre Fortsetzung, Figur 10a, zeigen die Herstellung der Hybridplasmide pS 200 bis 204, die synthetische CSF-DNA-Teilsequenzen enthalten, wobei das Plasmid pS 200 den "Syntheseblock I" gemäß Anhang I, das Plasmid pS 201 den "Syntheseblock II" gemäß Anhang II, das Plasmid pS 202 den "Syntheseblock III" gemäß Anhang III, das Plasmid pS 203 das gesamte synthetische Gen enthält und pS 204 ein Expressionsplasmid darstellt, das ebenfalls die gesamte synthetische CSF-DNA-Sequence enthält. Nach Expression und Säurespaltung wird das gleiche CSF-Derivat erhalten, das bei der Figur 2 beschrieben ist.

Die Figur 11 und ihre Fortsetzung, Figur 11a, zeigen die Synthese des Expressionsplasmids pS 207, das für ein Fusionsprotein codiert, das nach Spaltung mit N-Bromsuccinimid ein CSF-Derivat liefert, bei dem in den Positionen 13 und 122 jeweils Trp durch His ersetzt ist.

Die Figur 12 zeigt eine synthetische DNA-Teilsequenz, die die Herstellung eines CSF-Derivats erlaubt, bei dem in Position 100 Ile durch Thr ersetzt ist.

Die Figur 13 und ihre Fortsetzung, Figur 13a, zeigen die Synthese des Expressionsplasmids pS 210, das für ein Fusionsprotein codiert, welches nach Bromcyan-Spaltung ein CSF-Derivat ergibt, bei dem alle Methionin-Reste durch neutrale Aminosäuren ersetzt sind, nämlich in Position 36 durch Ile und in den Positionen 46, 79 und 80 durch Leu.

Die Figur 14 zeigt eine synthetische DNA-Sequenz, die nach dem Syntheseschema der Figur 13 die Herstellung eines CSF-Derivats erlaubt, bei dem in Position 36 Met durch Ile und in Position 46 Met durch Leu ersetzt ist und anstelle der Aminosäuren 79 und 80 ein einziger Leu-Rest steht.

Die Figur 15 zeigt schließlich eine synthetische DNA, deren Einsatz im Syntheseschema gemäß Figur 13 die Herstellung eines CSF-Derivats erlaubt, bei dem in Position 36 Met durch Ile und in Position 46 durch Leu ersetzt ist und wobei die beiden Aminosäuren in Position 79 und 80 deletiert sind.

Diese in den Figuren bzw. Beispielen ausgeführten Variationsmöglichkeiten sind selbstverständlich nur beispielhaft für die Vielzahl der Abwandlungen, die erfindungsgemäß möglich sind. So können in an sich bekannter Weise auch andere Proteinsequenzen, vor allem bakterielle, als "Ballast"-Anteil für die Fusionsproteine dienen und es können zur Verknüpfung und Spaltung der Fusionsproteine alle üblichen Methoden angewandt werden, wobei andere CSF-Derivate mit veränderter Aminosäurefolge im Molekül oder an den beiden Molekülenden resultieren können. Die Wahl der IL-2 und der synthetischen DNA-Sequenzen und die Spaltung der Fusionsproteine sind deshalb nur als bevorzugte Ausgestaltungen der Erfindung zu betrachten, die in an sich bekannter Weise variiert werden können.

Es hat sich gezeigt, daß der "offene Leseraster" aus einer DNA, die für Interleukin-2 codiert, als Expressionshilfe zur Expression von Peptiden bzw. Proteinen besonders vorteilhaft ist und daß ein N-terminaler Anteil von IL-2, der im wesentlichen den ersten 100 Aminosäuren entspricht, besonders gut zur Herstellung von Fusionsproteinen geeignet ist. Man erhält also als Primärprodukt ein Fusionsprotein, das völlig oder zum ganz überwiegenden Teil aus eukaryotischen Proteinsequenzen besteht. Überraschenderweise wird dieses Protein offenbar von den wirtseigenen Proteasen nicht als Fremdprotein erkannt und auch nicht sofort wieder abgebaut. Ein weiterer Vorteil ist, daß die erfindungsgemäßen Fusionsproteine schwer löslich bis unlöslich sind und sich somit einfach, zweckmäßig durch Zentrifugation, von den löslichen Proteinen abtrennen lassen.

Da es erfindungsgemäß hinsichtlich der Funktion als "Ballast-Anteil" des Fusionsproteins nicht darauf ankommt, daß der IL-2-Anteil ein biologisch aktives Molekül darstellt, kommt es insofern auch nicht auf die exakte Struktur des IL-2-Anteils an. Es genügt hierfür, daß im wesentlichen die ersten 100 N-terminalen Aminosäuren vorliegen. Es ist also beispielsweise möglich, am N-Terminus Variationen vorzunehmen, die eine Spaltung des Fusionsproteins erlauben, falls das erwünschte Protein N-terminal dazu angeordnet ist. Umgekehrt kann man C-terminal Variationen vornehmen, um die Abspaltung des gewünschten Proteins zu ermöglichen oder zu erleichtern.

Die für Human-IL-2 codierende natürliche DNA-Sequenz ist aus der europäischen Patentanmeldung mit der Veröffentlichungsnummer - in folgenden als "EP-A" bezeichnet - 0 091 539 bekannt. Die dort aufgeführte Literatur bezieht sich auch auf Mäuse - und Ratten-IL-2. Diese Säuger-DNA kann zur Synthese der erfindungsgemäßen Proteine herangezogen werden. Zweckmäßiger geht man jedoch von einer synthetischen DNA aus, besonders vorteilhaft von der DNA für Human-IL-2, die in der deutschen Offenlegungsschrift 3 419 995 bzw. in der EP-A 0 163 249 beschrieben wurde. Diese synthetische DNA hat nicht nur den Vorzug, daß sie in der Codon-Wahl auf die Gegebenheiten des am häufigsten verwendeten Wirts, E. coli, abgestimmt ist, sondern sie enthält auch eine Reihe von Schnittstellen für Restriktionsendonucleasen am Anfang bzw. in der Region des 100. Tripletts, von denen erfindungsgemäß Gebrauch gemacht werden kann. Hierdurch ist jedoch nicht ausgeschlossen, daß in dem dazwischenliegenden Bereich Variationen in der DNA vorgenommen werden, wobei von den weiteren Schnitt stellen Gebrauch gemacht werden kann.

Wird von den Nucleasen Ban II, Sac I oder Sst I Gebrauch gemacht, so erhält man eine Il-2-Teilsequenz, die für etwa 95 Aminosäuren codiert. Diese Länge ist im allgemeinen ausreichend, um ein unlösliches Fusionsprotein zu erhalten. Wenn die Schwerlöslichkeit, beispielsweise bei einem gewünschten hydrophilen CSF-Derivat, noch nicht ausreicht, man aber - um so wenig "Ballast" wie möglich zu produzieren - nicht von den näher am C-Terminus liegenden Schnittstellen Gebrauch machen will, so kann man durch entsprechende Adapter bzw. Linker die DNA-Sequenz am N-und/oder C-terminalen Ende verlängern und so den "Ballast"-Anteil "maßschneidern". Man kann natürlich auch die DNA-Sequenz - mehr oder weniger - bis zum Ende nutzen und so - gegebenenfalls modifiziertes - biologisch aktives IL-2 als "Nebenprodukt" erzeugen.

Die Spaltung des Fusionsproteins kann in an sich bekannter Weise chemisch oder enzymatisch erfolgen. Die Wahl der geeigneten Methode richtet sich vor allem nach der Aminosäuresequenz des gewünschten Proteins. Steht im Brückenglied Y am Carboxyterminus Tryptophan bzw. Methionin oder Y für Trp bzw. Met, so kann eine chemische Spaltung mit N-Bromsuccinimid bzw. Halogencyan erfolgen, sofern jeweils CSF-Derivate synthetisiert werden, die diese Aminosäuren nicht enthalten.

CSF und seine Derivate, die in ihrer Aminosäuresequenz

Asp - Pro

enthalten und hinreichend säurestabil sind, können, wie vorstehend schon gezeigt, in an sich bekannter Weise proteolytisch gespalten werden. Hierdurch erhält man Proteine, die N-terminal Prolin bzw. C-terminal Asparaginsäure enthalten. Auf diese Weise können also auch modifizierte Proteine synthetisiert werden.

Die Asp-Pro-Bindung kann noch säurelabiler gestaltet werden, wenn dieses Brückenglied $(Asp)_n$-Pro bzw. Glu-$(Asp)_n$-Pro ist, wobei n 1 bis 3 bedeutet.

Beispiele für enzymatische Spaltungen sind ebenfalls bekannt, wobei auch modifizierte Enzyme mit verbesserter Spezifität eingesetzt werden können (vgl. C.S. Craik et al., Science 228 (1985) 291-297).

Das Fusionsprotein wird durch Expression in einem bakteriellen Expressionssytem in an sich bekannter Weise gewonnen. Hierfür eignen sich alle bekannten Wirts-Vektor-Systeme wie Bakterien der Arten Streptomyces, B. subtilis, Salmonella typhimurium oder Serratia marcescens, insbesondere E. coli.

Die DNA-Sequenz, die für das gewünschte Protein codiert, wird in bekannter Weise in einen Vektor eingebaut, der in dem gewählten Expressionssytem eine gute Expression gewährleistet.

Hierbei wählt man zweckmäßig den Promoter und Operator aus der Gruppe trp, lac, tac, $P_L$ oder $P_R$ des Phagen λ, hsp, omp oder einen synthetischen Promoter, wie sie beispielsweise in der deutschen Offenlegungsschrift 34 30 683 bzw. in der EP-A 0 173 149 vorgeschlagen sind. Vorteilhaft ist die tac-Promoter-Operator-Sequenz, die inzwischen handelsüblich ist (z.B. Expressionsvektor pKK223-3, Pharmacia, "Molecular Biologicals, Chemicals and Equipment for Molecular Biology", 1984, S. 63).

Bei der Expression des erfindungsgemäßen Fusionsproteins kann es sich als zweckmäßig erweisen, einzelne Tripletts der ersten Aminosäuren nach dem ATG-Start-Codon zu verändern, um eine eventuelle Basenpaarung auf der Ebene der mRNA zu verhindern. Solche Veränderungen wie Deletionen oder Additionen einzelner Aminosäuren, sind dem Fachmann geläufig und ebenfalls Gegenstand der Erfindung.

Besonders vorteilhaft sind CSF-Derivate, die N-terminal Prolin enthalten, da solche Proteine stabiler gegen den Angriff von Proteasen sind. Besonders bevorzugt ist das CSF-Derivat, das im Anschluß an das N-terminal addierte Prolin die gesamte CSF-Aminosäuresequenz hat. Überraschenderweise hat es sich jedoch gezeigt, daß auch die Variationen des CSF-Moleküls, die durch Eliminierung der ersten 11 Aminosäuren erhalten werden, biologisch aktiv sind.

Vorteilhaft sind auch Varianten der Erfindung, die zunächst zu Fusionsproteinen führen, die die CSF-Sequenz mehr als einmal, vorteilhaft zwei- oder dreimal, enthalten. In diesen Fusionsproteinen ist naturgemäß der Ballast-Anteil reduziert und damit die Ausbeute des gewünschten Proteins erhöht.

Bei der American Type Culture Collection ist unter der Nummer ATCC 39900 in E. coli das Plasmid pHG 23 hinterlegt, das durch Einbau der CSF-cDNA-Sequenz in die Pst I-Schnittstelle von pBR 322 erhalten wurde. Die DNA-Sequenz entspricht hierbei der in Figur 3 (B) von Wong et al. beschriebenen Variante. Beim Einbau wurde von der Pst I-Schnittstelle nahe dem 5′-Ende einerseits und von einer durch GC-"Tailing" eingeführten Pst I-Stelle am 3′-Ende Gebrauch gemacht (EP-A 0 183 350).

## Beispiel 1

Direktexpression von CSF

Der handelsübliche Vektor pUC 12 wird mit den Restriktionsenzymen Sma I und Pst I geöffnet und das große Fragment (1) isoliert.

Aus der cDNA-Sequenz für CSF wird durch Schneiden mit den Enzymen Sfa NI und Pst I das Fragment (2) gewonnen, das mit dem synthetischen Linker (3) und anschließend mit dem pUC 12-Fragment (1) ligiert wird. Das so erhaltene Hybridplasmid pW 201 (4) enthält die CSF-DNA-Sequenz im Anschluß an das Startcodon ATG.

Das Hybridplasmid (4) wird mit Nco I geöffnet und die überstehenden Enden zum glattendigen Fragment (5) aufgefüllt. Der Vektor pUC 12 wird mit dem Enzym Eco RI geöffnet, worauf die überstehenden Enden aufgefüllt werden. Anschliessend erfolgt eine Behandlung mit Alkalischer Rinderphosphatase, wobei das pUC 12-Derivat (6) erhalten wird.

Durch Ligieren der Fragmente (5) und (6) erhält man Vektoren, die die CSF-DNA-Sequenz in beiden Orientierungen enthalten. Sie werden als pW 203 (7) bezeichnet.

Aus dem Vektor (7) wird mit Eco RI und Rsa I das Fragment (8) isoliert, das die Codons für die Aminosäuren 63 bis 127 von CSF enthält. Andererseits wird aus dem Vektor (4) durch Schneiden mit Nco I und Rsa I das Fragment (9) isoliert, das die Codons für die Aminosäuren 1 bis 61 von CSF enthält.

Das Plasmid pH 131/5 (Deutsche Offenlegungsschrift 35 14 113, bzw. EP-A 0 198 415, Beispiel 1, Figure 1) (10) wird mit Pvu II geöffnet, das kleine Fragment entfernt und das größere zum Plasmid pPH 160 (11) ligiert, das in E. coli-Zellen in höherer Kopienzahl vorliegt. Das Plasmid (11) wird mit Nco I und Eco RI geöffnet und das große Fragment (12) isoliert.

Die Fragmente (8), (9) und (12) werden nunmehr zu dem Hybridplasmid pW 206 (13) ligiert. Hierdurch wird das Codon für die Aminosäure 62 wiederhergestellt.

Das handelsübliche Plasmid pKK 65-10 (PL Biochemical Inc.) wird mit Eco RI gespalten und das Fragment (14) isoliert, das die beiden Terminatoren T 1 und T 2 enthält. Dieses Fragment (14) wird in das mit Eco RI geöffnete Plasmid (13) eingesetzt, wobei das Plasmid pW 225 (15) erhalten wird

E. coli 24-Bakterien, die das Plasmid (15) enthalten, werden in LB-Medium (J. H. Miller, Experiments in Molecular Genetics, Cold Spring Harbor Laboratory, 1972) mit 30 bis 50 µg/ml Ampicillin über Nacht bei 37 °C angezogen. Die Kultur wird mit M9-Medium (J. M. Miller, a.a.O.), das 2000 µm/l Casamino Acids und 1 µg/l Thiamin enthält, im Verhältnis 1:100 verdünnt und bei 37 °C unter ständiger Durchmischung inkubiert. Bei einer $OD_{600}$ = 0,5 bzw. 1 wird bis zu einer Endkonzentration von 15 µg/l Indolyl-3-acrylsäure zugesetzt und 2 bis 3 Stunden bzw. 16 Stunden inkubiert. Anschließend werden die Bakterien abzentrifugiert. Die Bakterien werden 5 Minuten in einer Puffermischung (7M Harnstoff, 0,1% SDS, 0,1 M Natriumphosphat, pH 7,0) gekocht und Proben auf eine SDS-Gelelektrophoreseplatte aufgetragen. Es zeigt sich, daß das Proteinmuster von Zellen, deren trp-Operon induziert wurde, im Bereich von etwa 14 000 - 18 000 Dalton ein neues Protein enthält, das bei nicht induzierten Zellen nicht gefunden wird.

Die angegebenen Induktionsbedingungen gelten für Schüttelkulturen; bei größeren Fermentationen sind entsprechend veränderte OD-Werte und gegebenenfalls leicht variierte Induktor-Konzentrationen zweckmäßig.

Beispiel 2

$Pro^0$-CSF

Der Vektor pUC 12 wird mit Eco RI und Pst I geöffnet und das große Fragment (16) isoliert. Dieses Fragment (16) wird mit dem synthetischen DNA-Fragment (17) und dem Fragment (2) (Beispiel 1; Figur 1) ligiert. Kompetente Zellen von E. coli JM 103 werden mit dem Ligationsgemisch transformiert und die gewünschten Klone selektiert, die das Plasmid pW 212 (18) enthalten.

Aus der Plasmid-DNA wird mit Pvu I und Pst I das Fragment (19) herausgespalten, das die CSF-Sequenz enthält.

Durch Insertion des lac-Repressors (P.J. Farabaugh, Nature 274 (1978) 765-769) in das Plasmid pKK 177-3 mit dem pUC 8-Polylinker (Amann et al., Gene 25 (1983) 167; EP-A 0 133 282) erhält man das Plasmid pJF118 (20) (Fig. 1; vgl. deutsche Patentanmeldung P 35 26 995.2, Beispiel 6, Fig. 6). Dieses wird an der singulären Restriktionsstelle für Ava I geöffnet und in an sich bekannter Weise durch Exonuclease-Behandlung um etwa 1000 bp verkleinert. Nach Ligierung wird das Plasmid pEW 1000 (21), erhalten, in dem das lac-Repressorgen vollständig erhalten ist, das aber auf Grunde der Verkleinerung in deutlich höherer Kopienzahl als das Ausgangsplasmid vorliegt.

Anstelle des Plasmids pKK177-3 kann man auch von dem vorstehend erwähnten handelsüblichen Plasmid pKK223-3 ausgehen, den lac-Repressor einbauen und das erhaltene Produkt analog verkürzen.

Das Plasmid pEW 1000 (21) wird mit den Restriktionsenzymen EcoR I und Sal I geöffnet und das Fragment (22) isoliert.

Das Plasmid p159/6 (23), hergestellt gemäß deutscher Offenlegungsschrift 3 419 995 (EP-A 0 163 249), Beispiel 4 (Figur 5), wird mit den Restriktionsenzymen Eco RI und Sal I geöffnet und das kleine Fragment (24) isoliert, das die IL-2-Sequenz enthält.

Durch Ligieren der Fragmente (22) und (24) erhält man das Hybridplasmid pEW 1001 (25).

Das Plasmid (25) wird einerseits mit Eco RI und Pvu I geöffnet, wobei man das Fragment (26) erhält, welches den größten Teil der IL-2-Sequenz enthält. Diese Teilsequenz wird in den Figuren mit "ΔIL2" bezeichnet.

Andererseits wird das Plasmid (25) mit Eco RI und Pst I geöffnet und das große Fragment (27) isoliert.

Durch Ligieren der Fragmente (19), (26) und (27), Transformation von kompetenten E. coli 294-Zellen und Selektion erhält man Klone, die das Plasmid pW 216 (28) enthalten. Die Plasmid-DNA wird durch Restriktions- und DNA-Sequenzanalyse charakterisiert.

Eine Übernachtkultur aus E. coli-Zellen, die das Plasmid (28) enthalten, wird mit LB-Medium (J. H. Miller, a.a.O), das 50 µg/ml Ampicillin enthält, im Verhältnis von etwa 1:100 verdünnt und das Wachstum über OD-Messung verfolgt. Bei OD = 0,5 wird die Kultur auf 1 mM Isopropyl-β-galactopyranosid (IPTG) eingestellt und die Bakterien nach 150 bis 180 Minuten abzentrifugiert. Die Bakterien werden 5 Minuten in einer Puffermischung (7M Harnstoff, 0,1% SDS, 0,1 M Natriumphosphat, pH 7,0) gekocht und Proben auf eine SDS-Gelelektrophoreseplatte aufgetragen. Nach Elektrophorese wird aus Bakterien, die das Plasmid (28) enthalten, eine Proteinbande erhalten, die der Größe des erwarteten Fusionsproteins entspricht. Nach Aufschluß der Bakterien (French Press; (R)Dyno-Mühle) und Zentrifugation befindet sich das Fusionsprotein im Niederschlag, so daß mit dem Überstand bereits erhebliche Mengen der übrigen Proteine abgetrennt werden können. Nach Isolierung des Fusionsproteins wird durch Säurespaltung das erwartete CSF-Derivat, das N-terminal zusätzlich Prolin enthält, freigesetzt. Dieses zeigt im biologischen Test Aktivität.

Die angegebenen Induktionsbedingungen gelten für Schüttelkulturen; bei größeren Fermentationen sind entsprechend veränderte OD-Werte und gegebenenfalls leicht variierte IPTG-Konzentrationen zweckmäßig.

## Beispiel 3

Pro$^1$-CSF(2-127)

Durch Ligierung der Fragmente (2) (Figur 1) und (16) (Figur 2) mit der synthetischen DNA-Sequenz (29) erhält man das Hybridplasmid (30), das bis auf die synthetische DNA-Sequenz dem Plasmid (18) entspricht.

Aus dem Plasmid (30) wird mit Pvu I und Pst I das Fragment (31) herausgespalten, das die CSF-DNA-Sequenz enthält, bei der jedoch das Codon für die erste Aminosäure durch ein Codon für Prolin ersetzt ist. Durch Ligieren des Fragments (31) mit den Fragmenten (26) und (27) erhält man das Hybridplasmid pW 240 (32). Die Expression in E. coli, die gemäß Beispiel 2 durchgeführt wird, liefert ein CSF-Derivat, in dem die erste Aminosäure durch Prolin ersetzt ist. Auch dieses Derivat zeigt biologische Aktivität.

## Beispiel 4

CSF(2-127)

Ein Plasmid, das die CSF-DNA-Sequenz mit einer Pst-I-Restriktionsstelle an deren 3′-Ende enthält, beispielsweise das Plasmid pHG 23 (ATCC 39900), wird mit Sfa NI gespalten und das linearisierte Plasmid (34) mit Hilfe von Klenow-Polymerase und GTP partiell aufgefüllt. Mit S1-Nuclease wird das überstehende Nucleotid A abgespalten und anschließend mit Pst I das Fragment (35) herausgespalten.

Durch Ligieren des Fragments (35) mit der synthetischen DNA-Sequenz (36) und dem Fragment (16) (Figur 2) erhält man das Plasmid (37), das analog zum Plasmid (18) ist.

Aus dem Plasmid (37) wird mit Pvu I und Pst I das Fragment (38) herausgespalten. Dieses Fragment wird mit den Fragmenten (26) und (27) ligiert, wodurch das Plasmid pW 241 (39) erhalten wird.

Durch Expression gemäß Beispiel 2 erhält man ein Fusionsprotein, das nach Säurespaltung ein CSF-Derivat liefert, bei dem die erste Aminosäure fehlt. Dieses Derivat ist biologisch aktiv.

## Beispiel 5

CSF(6-127)

Das Plasmid (33) (oder ein entsprechendes Plasmid, das die CSF-DNA-Sequenz enthält) wird zunächst mit Pst I total und anschließend mit Bst NI partiell gespalten und das Fragment (40) isoliert.

Die synthetischen DNA-Sequenzen (41) und (36) (Figur 4) werden zunächst zur Sequenz (42) und diese dann mit dem Fragment (16) (Figur 2) und dem Fragment (40) ligiert, wobei das Plasmid pW 212 (43) erhalten wird.

Aus dem Plasmid (43) wird mit Pvu I und Pst I das Fragment (44) isoliert, das die DNA-Sequenz für das CSF-Derivat enthält. Dieses Fragment (44) wird mit den Fragmenten (26) und (27) ligiert, was zum Hybridplasmid pW 242 (45) führt.

Die Expression gemäß Beispiel 2 führt zu einem Fusionsprotein, aus dem nach Säurespaltung ein CSF-Derivat erhalten wird, bei dem die ersten 5 Aminosäure fehlen. Auch dieses Produkt ist biologisch aktiv.

Beispiel 6

CSF(8-127)

Ligiert man zunächst die synthetische DNA-Sequenz (36) (Figur 4) mit der synthetischen DNA-Sequenz (46) und anschließend das so erhaltene DNA-Fragment (47) mit den Fragmenten (40) und (16), so erhält man das Hybridplasmid (48). Aus diesem wird mit Pvu I und Pst I das Fragment (49) herausgespalten, das die DNA-Sequenz für das CSF-Derivat enthält. Die Ligation der Fragmente (49), (26), und (27) liefert das Hybridplasmid pW 243 (50), das dem Plasmid (45) bis auf die verkürzte DNA-Sequenz für das CSF-Derivat entspricht.

Die Expression nach Beispiel 2 führt zu einem Fusionsprotein, das nach Säurespaltung ein CSF-Derivat ergibt, bei dem die ersten 7 Aminosäuren fehlen. Auch dieses Derivat ist biologisch aktiv.

Beispiel 7

CSF(12-127)

Ligiert man die synthetische DNA-Sequenz (51) mit den Fragmenten (33) und (16), so erhält man das Hybridplasmid (52). Spaltet man aus diesem mit Pvu I und Pst I die Sequenz (53) heraus, die die DNA-Sequenz für das CSF-Derivat enthält, und ligiert dieses Fragment (53) mit den Fragmenten (26) und (27), so erhält man das Hybridplasmid pW 244 (54), das bis auf die verkürzte CSF-Sequenz dem Plasmid (45) entspricht.

Die Expression gemäß Beispiel 2 führt zu einem Fusionsprotein, das nach Säurespaltung ein CSF-Derviat liefert, bei dem die Aminosäure 1 bis 11 eliminiert sind. Auch dieses verkürzte Molekül ist biologisch aktiv.

Beispiel 8

$Pro^0$-CSF(1-126)-Asp

Man spaltet die DNA-Sequenz (19) (Figur 2) partiell mit Bst NI und isoliert das Fragment (55), das den größten Teil der CSF-Sequenz enthält.

Durch Spaltung des Plasmids (33) (Figur 4) (oder eines entsprechenden Plasmids, das die CSF-DNA-Sequenz enthält) mit zunächst Pst I und anschließend partiell mit Bst NI erhält man die DNA-Sequenz (56), die den größten Teil der CSF-Sequenz umfaßt.

Man synthetisiert die DNA-Sequenz (57), die die Sequenz (56) zu einer DNA-Sequenz ergänzt, die für ein CSF-Derivat codiert, in welchem C-terminal Glutaminsäure durch Asparaginsäure ersetzt ist.

Der Vektor pUC 13 wird mit Pst I und Sma I geöffnet und das große Fragment (58) isoliert. Ligiert man dieses Restplasmid (58) mit den Fragmenten (56) und (57), so erhält man das Hybridplasmid pW 245 (59) mit der C-terminal modifizierten Sequenz.

Aus dem Plasmid (59) wird mit Sfa NI und Pst I das Fragment (60) herausgespalten, das die modifizierte CSF-DNA-Sequenz enthält. Dieses Fragment (60) wird mit der synthetischen DNA-Sequenz (61) und dem Fragment (55) ligiert, wobei die DNA-Sequenz (62) erhalten wird. Diese wird mit den DNA-Fragmenten (26) und (27) (Figur 2) ligiert, wobei das Hybridplasmid pW 246 (63) erhalten wird. Dieses Plasmid ist in der Figur 8a zweimal wiedergegeben, wobei aus der unteren Darstellung die Aminosäuresequenz des codierten Fusionsproteins zu entnehmen ist.

Die Expression gemäß Beispiel 2 ergibt ein Fusionsprotein, aus dem nach Säurespaltung ein CSF-Derivat hervorgeht, das N-terminal um Prolin verlängert ist und in dem zusätzlich die letzte Aminosäure durch Asparaginsäure ersetzt ist. Dieses Derivat ist biologisch aktiv.

Beispiel 9

Pro$^0$-CSF(1-126)-Asp

Man spaltet das Hybridplasmid (63) (Figur 8) mit Eco RI und Pst I und isoliert das Fragment, das im Anschluß an die IL-2-Teilsequenz die beiden modifizierten CSF-Sequenzen enthält. Diese Sequenz (64) wird mit Rsa I partiell gespalten und die beiden Fragmente (65) und (66) isoliert. Durch Ligieren der DNA-Sequenzen (27), (65), (67), (61) und (60) erhält man das Hybridplasmid pW 247 (68), in dem die ligierten Sequenzen in der genannten Reihenfolge angeordnet sind.

Die Expression nach Beispiel 2 liefert ein Fusionsprotein, aus dem nach Säurespaltung dasselbe CSF-Derivat wie nach Beispiel 8 resultiert.

## Beispiel 10

Synthetisches Gen (für Pro$^0$-CSF)

Nach an sich bekannten Verfahren, beispielsweise nach der Phosphit-Methode (Deutsche Offenlegungsschriften 3 327 007, 3 328 793, 3 409 966, 3 414 831 und 3 419 995) werden die drei "Syntheseblocks" I (CSF-I), in den Figuren als (69) bezeichnet, II (CSF-II), in den Figuren (70), und III (CSF-III), in den Figuren (71), synthetisiert. In der Nucleotidfolge dieser Syntheseblocks sind die synthetisierten Oligonucleotide Ia bis Im, IIa bis IIf und IIIa bis IIII eingezeichnet (Anhang).

Bei der Auswahl der Nucleotide für das synthetische Gen wurden nicht nur singuläre Schnittstellen an den Nahtstellen der drei Syntheseblocks, sondern auch innerhalb der Genfragmente eine Reihe von singulären Restriktionsstellen vorgesehen. Diese sind in den folgenden Tabellen aufgeführt. Diese singulären Restriktionsstellen können in an sich bekannter Weise dazu dienen, Codons für Aminosäuren auszutauschen, zu ergänzen oder zu deletieren.

### Syntheseblock I (CSF I)

| Enzym | Erkennungssequenz | Schnitt nach Nucleotid Nr. (codierender Strang) |
|---|---|---|
| Nar I | GG↓CGCC | 1 |
| Hpa II | C↓CGG | 4 |
| Hae II | GGCGC↓C | 4 |
| Nae I | GCC↓GGC | 5 |
| Pvu I | CGAT↓CG | 13 |
| Sal I | G↓TCGAC | 24 |
| Acc I | GT↓CGAC | 25 |
| Hinc II | GTC↓GAC | 26 |
| Hpa I/ Hinc II | GTT↓AAC | 48 |
| Hha I | GCG↓C | 66 |
| Hinf I | G↓AGTC | 88 |
| Nru I | TCG↓CGA | 89 |
| Xma III | C↓GGCCG | 95 |
| Sac II | CCGC↓CG | 101 |
| Eco RV | GAT↓ATC | 128 |

### Syntheseblock II (CSF-II)

| Enzym | Erkennungssequenz | Schnitt nach Nucleotid Nr. (codierender Strang) |
|---|---|---|
| Afl III | A↓CATGT | 157 |
| Mlu I | A↓CGCGT | 169 |
| Xho I | C↓TCGAC | 175 |
| Taq I | T↓CGA | 176 |
| Hga I | GACGC (5/10) | 177 |
| Ava I | C↓TCGAG | 177 |
| Alu I | AG↓CT | 180 |
| Sac I/ Hgi AI | GAGCT↓C | 182 |
| Stu I/ Hae I | AGG↓CCT | 194 |

Syntheseblock III (CSF-III)

| Enzym | Erkennungssequenz | Schnitt nach Nucleotid Nr. (codierender Strang) |
|---|---|---|
| Afl II | C↓TTAAG | 217 |
| Hae III | GG↓CC | 224 |
| Apa I | GGGCC↓C | 227 |
| Mnl I | CCTC (7/7) | 238 |
| Nhe I | G↓CTAGC | 241 |
| Mae I | C↓TAG | 242 |
| Aha II | GA↓CGTC | 280 |
| Aat II | GACGT↓C | 283 |
| Sci NI | G↓CGC | 287 |
| Mst I | TCG↓GCA | 288 |
| Sau 3AI/ Mbo I | ↓GATC | 296 |
| Dpn I | GA↓TC | 298 |
| Asu II | TT↓CGAA | 308 |
| Aha III | TTT↓AAA | 318 |
| Ava II | G↓GTCC | 382 |
| Eco RII | ↓CCAGG | 384 |
| Bst NI/ Scr FI | CC↓AGG | 380 |

Die drei Syntheseblocks wurden zunächst einzeln kloniert, in E. coli amplifiziert und reisoliert:

Der Syntheseblock CSF-I (69) wird in das pUC 12-Derivat (16) eingebaut, wobei das Plasmid pS 200 (72) erhalten wird.

pUC 12 wird mit den Restriktionsenzmyen Pst I und Hind III geöffnet und mit dem linearisierten Plasmid (73) der Syntheseblock CSF-II (70) ligiert, wobei das Plasmid pS 201 (74) erhalten wird.

pUC 13 wird mit Hind III und Sma I geöffnet und das linearisierte Plasmid (75) mit CSF-III (71) ligiert, wobei das Plasmid pS 202 (76) erhalten wird.

Die reisolierten Syntheseblocks (69), (70) und (71) werden nunmehr in den mit Eco RI und Sma I linearisierten Vektor pUC 12 (77) ligiert, wobei das Plasmid pS 203 (78) resultiert. Dieses Hybridplasmid wird - wie die Plasmide mit den einzelnen Syntheseblocks - in E. coli 79/02 amplifiziert und das synthetische Gen durch Restriktions- und Sequenzanalyse charakterisiert.

Das Plasmid (78) wird mit Pvu I partiell und Bam HI gespalten und das kleine Fragment (79) mit der gesamten CSF-Sequenz isoliert.

Das Expressionsplasmid (21) wird mit Eco RI und Bam HI geöffnet und das große Fragment (80) isoliert. Dieses Fragment (80) wird nunmehr mit dem Fragment (26), das die IL-2-Teilsequenz enthält, und dem synthetischen Gen (79) ligiert. Hierbei wird das Plasmid pS 204 (81) erhalten, das für ein Fusionsprotein codiert, in welchem auf die IL-2-Teilsequenz zunächst das Brückenglied folgt, welches die Säurespaltung erlaubt, und anschließend die Aminosäuresequenz von CSF. Durch Säurespaltung resultiert somit ein CSF-Derivat, das N-terminal um Prolin verlängert ist.

Beispiel 11

CSF(1-12)His(14-121)His(123-127)

Ersetzt man im Syntheseblock I die Oligonucleotide Ia und Ib sowie Ic und Id durch die synthetischen Sequenzen (82) und (83), so erhält man einen modifizierten Syntheseblock I, der für ein CSF-I-Analoges codiert, in dem vor der ersten Aminosäure (Ala) Trp steht und in Position 13 Trp durch His ersetzt wird.

Das Plasmid (72) (Figur 10) wird mit Eco RI und Hpa I geöffnet und das große Fragment (84) isoliert. Dieses wird nun mit den synthetischen Fragmenten (82) und (83) ligiert, wobei das Plasmid pS 205 (85) erhalten wird, das für dieses modifizierte CSF I (CSF-I′) codiert.

Man öffnet das Plasmid (76) (Figur 10) mit Hind III und Sal I und isoliert das kleine (86) und große (87) Fragment. Das kleine Fragment (86) wird mit Taq I nachgeschnitten und das Fragment (88) isoliert.

Das große Fragment (87) wird nunmehr mit (88) und dem synthetischen Fragment (89), in dem in Position 122 das Codon für Trp durch His ersetzt ist, ligiert, wobei man das Plasmid pS 206 (90) erhält, das für das modifizierte CSF III (CSF-III′) codiert. Dieses Plasmid wird nach E. coli transformiert, amplifiziert, reisoliert, mit Hind III und Sal I geschnitten und das kleine Fragment (91), das für CSF-III′ codiert, isoliert.

Das Plasmid (85) wird mit Pvu I partiell und mit Pst I geschnitten und das kleine Fragment (92), das für CSF-I′ codiert, isoliert.

Ligiert man nunmehr die Fragmente (22), (26), (92), (70) und (91), so erhält man das Plasmid pS 207 (93). Dieses codiert für ein Fusionsprotein, in welchem auf die IL-2-Teilsequenz ein Brückenglied folgt, das unmittelbar vor der ersten Aminosäure von CSF (Ala) Trp enthält. Da in dem CSF-Molekül Trp in den Positionen 13 und 122 durch His ersetzt wurde, kann nunmehr eine Spaltung des Fusionsproteins mit N-Bromosuccinimid erfolgen. Hierbei erhält man das CSF-Derivat, in dem in beiden Positionen Tryptophan durch Histidin ersetzt ist.

Beispiel 12

CSF(1-99)Thr(101-127)

Ersetzt man beim Aufbau des Syntheseblocks III die Oligonucleotide IIIe und IIIf durch die synthetische Sequenz (94) und verfährt im übrigen gemäß Beispiel 10, so erhält man ein CSF-Derivat, in dem in Position 100 Ile durch Thr ersetzt ist.

Beispiel 13

CSF(1-35)Ile(37-45)Leu(47-78)Leu-Leu(81-127)

Man synthetisiert zunächst das Oligonucleotid (95), das in Position 36 das Codon für Ile anstelle von Met enthält, und das Oligonucleotid (96), in welchem in Position 46 das Codon für Met durch Leu ersetzt ist.

Anschließend wird das Plasmid (72) (Figur 10) mit Pvu I und Xma III geöffnet und das Fragment (97) isoliert.

Weiterhin synthetisiert man die Sequenz (98), bei der vor dem Codon für die erste Aminosäure das Codon für Met angeordnet ist.

Ligiert man nun die Fragmente (16), (98), (97), (95) und (96), so erhält man das Plasmid pS 208 (99). Dieses entspricht dem Plasmid (72), enthält jedoch in der CSF-I-Sequenz in Position O das Codon für Met, in Position 36 ein Codon für Ile und in Position 46 ein Codon für Leu.

Man synthetisiert weiterhin die Sequenz (100), die in den Positionen 79 und 80 für Leu anstelle von Met codiert.

Öffnet man das Plasmid (76) (Figur 10) mit Hind III und Nhe I, isoliert das große Fragment (101) und ligiert es mit der synthetischen Sequenz (100), so erhält man das Plasmid pS 209 (102), das dem Plasmid (76) bis auf den Austausch der beiden Codons in Position 79 und 80 in der CSF-III-Sequenz entspricht.

Man schneidet nunmehr das Plasmid (93) (Figur 11a) partiell mit Pvu I und mit Sal I und isoliert das große Fragment (103). Das Plasmid (99) wird ebenfalls partiell mit Pvu I und mit Pst I geöffnet und das kleine Fragment (104) isoliert, das die modifizierte CSF-I-Sequenz enthält. Ausserdem öffnet man das Plasmid (102) mit Hind III und Sal I und isoliert das kleine Fragment (105), das die modifizierte CSF-III-Sequenz umfaßt.

Nunmehr ligiert man die Fragmente (103), (104), (70) und (105), wobei das Plasmid pS 210 (106) erhalten wird, das dem Plasmid (93) (Figur 11a) entspricht, jedoch für ein CSF-Derivat codiert, bei dem in Position O Met steht und andererseits die vier Met-Reste durch andere Aminosäuren ersetzt sind.

Transformiert man mit dem Plasmid (106) E. coli, so erhält man nach Induktion ein Fusionsprotein, das mit Halogencyan spaltbar ist, wobei ein CSF-Derivat erhalten wird, das in Position 36 Ile und in den Positionen 46, 79 und 80 Leu enthält.

Beispiel 14

CSF(1-35)Ile(37-45)Leu(47-78)Leu(81-127)

Verfährt man gemäß Beispiel 13, verwendet jedoch anstelle der synthetischen Sequenz (100) die synthetische Sequenz (107), so erhält man ein Deletionsprodukt, bei dem in Position 36 Ile und in Position 46 Leu steht und wobei anstelle der Aminosäuren 79 und 80 die Aminosäure Leu vorkommt.

Beispiel 15

CSF(1-35)Ile(37-45)Leu(47-78)-(81-127)

Verfährt man gemäß Beispiel 13, setzt jedoch anstelle der synthetischen Sequenz (100) die synthetische Sequenz (108) ein, so erhält man ein Deletionsprodukt, bei dem in Position 36 Ile und in Position 46 Leu steht und die Aminosäuren in Position 79 und 80 deletiert sind.

ANHANG

Syntheseblock I (CSF-I) (69)

```
←————————Ia————————→←——————————Ic
                      1          •          •
AAT TCG ATC GAC GAC CCG GCG CCG GCC|CGA TCG CCG TCT CCG
     GC TAG CTG CTG GGC CGC GGC CGG GCT AGC|GGC AGA GGC
(Eco RI)  Ile Asp Asp Pro Ala Pro Ala Arg Ser Pro Ser Pro
     ←————————Ib———(1)——————————(5)→←——————Id

Ic————————→←—————Ie———50————————————→←——Ig
      •          •          •          •
TCG ACC CAG CCC|TGG GAA CAC GTT AAC GCG ATC CAG GA|A GCG
AGC TGG GTC GGG ACC CTT|GTG CAA TTG CGC TAG GTC CTT CGC
Ser Thr Gln Pro Trp Glu His Val Asn Ala Ile Gln Glu Ala
Id———(10)———————————→←(15)——————————(20)——————————If

Ig————————————————————————→←————————100————————Ii
      •          •          •          •
 CGG CGT CTG CTG AAC CTG AGT CGC|GAC ACG GCC GCG GAA ATG
 GC|C GCA GAC GAC TTG GAC TCA GCG CTG TGC|CGG CGC CTT TAC
 Arg Arg Leu Leu Asn Leu Ser Arg Asp Thr Ala Ala Glu Met
If→←————(25)————————Ih————(30)————————→←————(35)————————Ik

--Ii————————————————→←——————————Il————————————149→
      •          •          •          •          •
AAC GAA ACC GTT GAA|GTG ATA TCT GAG ATG TTC GAC CTG CA
TTG CTT TGG CAA CTT CAC TAT|AGA CTC TAC AAG CTG G (Pst I)
Asn Glu Thr Val Glu Val Ile Ser Glu Met Phe Asp(Leu)
--Ik——————————(40)——————————→←——(45)——————Im——→    (50)
```

Syntheseblock II (CSF-II) (70)

150
IIa IIc

(Pst I)G GAA CCG ACA TGT CTC CAG ACG CGT CTC GAG CTC TAC
AC GTC CTT GGC TGT ACA GAG GTC TGC GCA GAG CTC GAG ATG
(Gln)Glu Pro Thr Cys Leu Gln Thr Arg Leu Glu Leu Tyr
(50) (55) (60)
IIb IId

IIc 200 IIe 214

AAA CAA GGC CTT CGT GGT TCT CTG ACC A (Hind III)
TTT GTT CCG GAA GCA CCA AGA GAC TGG TTC GA
Lys Gln Gly Leu Arg Gly Ser Leu Thr(Lys)
(65) (70)
IId IIf

Syntheseblock III (CSF-III) (71)

```
215                    IIIa                      250      IIIc
 AG CTT AAG GGG CCC CTC ACC ATG ATG GCT AGC CAC|TAC AAA
(Hind III)A TTC CCC GGG GAG TGG TAC TAC CGA TCG GTG ATG TTT|
    (Leu)Lys Gly Pro Leu Thr Met Met Ala Ser His Tyr Lys
  (72)       (75)        IIIb     (80)            (85)

IIIc                                              IIIe
CAG CAC TGC CCG CCG ACT CCG GAG ACG|TCT TGC GCA ACG CAG
GTC GTG ACG GGC GGC TGA GGC CTC TGC AGA ACG|CGT TGC GTC
Gln His Cys Pro Pro Thr Pro Glu Thr Ser Cys Ala Thr Gln
                (90)  IIId        (95)            IIIf

IIIe 300                  IIIg
ATC ATC|ACC TTC GAA TCT TTT AAA GAA AAC CTG AAG GAC TTT
TAG TAG TGG AAG|CTT AGA AAA TTT CTT TTG GAC TTC CTG AAA
Ile Ile Thr Phe Glu Ser Phe Lys Glu Asn Leu Lys Asp Phe
IIIf (100)         (105)               (110)         IIIh

             350   IIIi                          391 IIIk
|CTG CTT GTT ATA CCG TTC GAC TGT TGG GAG|CCG GTC CAG GAA
GAC GAA|CAA TAT GGC AAG CTG ACA ACC CTC GGC CAG|GTC CTT
Leu Leu Val Ile Pro Phe Asp Cys Trp Glu Pro Val Gln Glu
IIIh (115)            (120)    IIIj        (125)     IIIl

IIIk
     Sal I   Pst I
TGA TAG TCG ACT GCA GCC C
ACT ATC AGC TGA CGT CGG G
Stp Stp            (Sma I)
IIIl
```

## Patentansprüche

### Patentansprüche für folgende Vertragsstaaten : BE, CH, DE, FR, GB, IT, LI, LU, NL, SE

**1.** Humane Granulozyten-Makrophagen-"Colony Stimulating"-Faktor-Proteine (GM-CSF) der Formel

Pro-$(As)_x$-CSF(12-126)-Z,

in der $(As)_x$ ganz oder teilweise die ersten 11 Aminosäuren der natürlichen GM-CSF-Sequenz und Z Glu oder Asp bedeuten, im folgenden "CSF".

**2.** Verfahren zur Herstellung von CSF gemäß Anspruch 1, dadurch gekennzeichnet, daß man ein für CSF codierendes Gen in einen bakteriellen Expressionsvektor einbaut, damit Bakterien, insbesondere E. coli, transformiert und darin zur Expression bringt.

**3.** Verfahren nach Anspruch 2, dadurch gekennzeichnet, daß man das CSF in Form eines Fusionsproteins exprimiert, das anschließend enzymatisch oder chemisch gespalten wird.

**4.** Verfahren nach Anspruch 3, dadurch gekennzeichnet, daß das Fusionsprotein N-terminal benachbart zum CSF die Aminosäuresequenz

$(Glu)_m$-$(Asp)_n$-Pro,

in der m Null oder 1 und n 1, 2 oder 3 ist, enthält und proteolytisch gespalten wird.

**5.** Bakterieller Expressionsvektor, enthaltend mindestens ein für CSF gemäß Anspruch 1 codierendes Gen.

**6.** Bakterienzelle, insbesondere E. coli, enthaltend einen Vektor nach Anspruch 5.

**7.** Arzneimittel, enthaltend oder bestehend aus CSF gemäß Anspruch 1.

**8.** CSF gemäß Anspruch 1 zur Anwendung in Heilverfahren.

**9.** Verwendung von CSF gemäß Anspruch 1 zur Herstellung von Arzneimitteln.

**Patentansprüche für folgende Vertragsstaaten : ES, AT**

**1.** Verfahren zur Herstellung von humanen Granulozyten-Makrophagen-"Colony Stimulating"-Faktor-Proteinen (GM-CSF) der Formel

Pro-$(As)_x$-CSF(12-126)-Z,

in der $(As)_x$ ganz oder teilweise die ersten 11 Aminosäuren der natürlichen GM-CSF-Sequenz und Z Glu oder Asp bedeuten, dadurch gekennzeichnet, daß man ein für CSF codierendes Gen in einen bakteriellen Expressionsvektor einbaut, damit Bakterien transformiert und darin zur Expression bringt.

**2.** Verfahren nach Anspruch 1, dadurch gekennzeichnet, daß das Bakterium E. coli ist.

**3.** Verfahren nach Anspruch 1 oder 2, dadurch gekennzeichnet, daß man das CSF in Form eines Fusionsproteins exprimiert, das anschließend enzymatisch oder chemisch gespalten wird.

**4.** Verfahren nach Anspruch 3, dadurch gekennzeichnet, daß das Fusionsprotein N-terminal benachbart zum CSF die Aminosäuresequenz

$(Glu)_m$-$(Asp)_n$-Pro,

in der m Null oder 1 und n 1, 2 oder 3 ist, enthält und proteolytisch gespalten wird.

**Patentansprüche für folgenden Vertragsstaat : GR**

**1.** Verfahren zur Herstellung von humanen Granulozyten-Makrophagen-"Colony Stimulating"Faktor-Proteinen (GM-CSF) der Formel

Pro-$As)_x$-CSF( 12-126)-Z,

in der $(As)_x$ ganz oder teilweise die ersten 11 Aminosäuren der natürlichen GM-CSF-Sequenz und Z Glu oder Asp bedeuten, dadurch gekennzeichnet, daß man ein für CSF codierendes Gen in einen bakteriellen Expressionsvektor einbaut, damit Bakterien transformiert und darin zur Expression bringt.

**2.** Verfahren nach Anspruch 1, dadurch gekennzeichnet, daß das Bakterium E. coli ist.

**3.** Verfahren nach Anspruch 1 oder 2, dadurch gekennzeichnet, daß man das CSF in Form eines Fusionsproteins exprimiert, das anschließend enzymatisch oder chemisch gespalten wird.

**4.** Verfahren nach Anspruch 3, dadurch gekennzeichnet, daß das Fusionsprotein N-terminal benachbart zum CSF die Aminosäuresequenz

$(Glu)_m$-$(Asp)_n$-Pro,

in der m Null oder 1 und n 1, 2 oder 3 ist, enthält und proteolytisch gespalten wird.

**5.** Bakterieller Expressionsvektor, enthaltend mindestens ein für CSF codierendes Gen gemäß Anspruch 1.

**6.** Bakterienzelle, enthaltend einen Vektor nach Anspruch 5.

**7.** E. coli, enthaltend einen Vektor nach Anspruch 5.

**8.** CSF gemäß Anspruch 1 zur Anwendung in Heilverfahren.

**9.** Verwendung von CSF gemäß Anspruch 1 Herstellung von Arzneimitteln.

**Claims**

**Claims for the following Contracting States : BE, CH, DE, FR, GB, IT, LI, LU, NL, SE**

**1.** Human granulocyte macrophage colony-stimulating factor proteins (GM-CSF) of the formula Pro-$(As)_x$-CSF(12-126)-Z in which $(AS)_x$ denotes all or some of the first 11 amino acids of the natural GM-CSF sequence, and Z denotes Glu or Asp, hereinafter referred to as CSF.

**2.** A process for the preparation of CSF as claimed in claim 1, which comprises incorporation of a gene coding for CSF in a bacterial expression vector, transformation of bacteria, especially E. coli, therewith, and bringing about expression therein.

**3.** The process as claimed in claim 2, wherein the CSF is expressed in the form of a fusion protein which is then cleaved enzymatically or chemically.

**4.** The process as claimed in claim 3, wherein the fusion protein contains N-terminal adjacent to the CSF the amino acid sequence

$(Glu)_m$-$(Asp)_n$-Pro

in which m is zero or 1, and n is 1, 2 or 3, and is proteolytically cleaved.

**5.** A bacterial expression vector containing at least one gene coding for CSF as claimed in claim 1.

**6.** A bacterial cell, especially E. coli, containing a vector as claimed in claim 5.

**7.** A medicament containing or composed of CSF as claimed in claim 1.

**8.** CSF as claimed in claim 1 for use in medical treatment.

**9.** The use of CSF as claimed in claim 1 for the preparation of medicaments.

**Claims for the following Contracting States : ES, AT**

1. A process for the preparation of human granulocyte macrophage colony-stimulating factor proteins (GM-CSF) of the formula

   Pro-$(As)_x$-CSF(12-126)-Z

   in which $(AS)_x$ denotes all or some of the first 11 amino acids of the natural GM-CSF sequence, and Z denotes Glu or Asp, which comprises incorporation of a gene coding for CSF in a bacterial expression vector, transformation of bacteria therewith, and bringing about expression therein.

2. The process as claimed in claim 1, wherein the bacterium is E. coli.

3. The process as claimed in 1 or 2, wherein the CSF is expressed in the form of a fusion protein which is then cleaved enzymatically or chemically.

4. The process as claimed in claim 3, wherein the fusion protein contains N-terminal adjacent to the CSF the amino acid sequence

   $(Glu)_m$-$(Asp)_n$-Pro

   in which m is zero or 1, and n is 1, 2 or 3, and is proteolytically cleaved.

**Claims for the following Contracting State : GR**

1. A process for the preparation of human granulocyte macrophage colony-stimulating factor proteins (GM-CSF) of the formula

   Pro-$(As)_x$-CSF(12-126)-Z

   in which $(AS)_x$ denotes all or some of the first 11 amino acids of the natural GM-CSF sequence, and Z denotes Glu or Asp, which comprises incorporation of a gene coding for CSF in a bacterial expression vector, transformation of bacteria therewith, and bringing about expression therein.

2. The process as claimed in claim 1, wherein the bacterium is E. coli.

3. The process as claimed in 1 or 2, wherein he CSF is expressed in the form of a fusion protein which is then cleaved enzymatically or chemically.

4. The process as claimed in claim 3, wherein the fusion protein contains N-terminal adjacent to the CSF the amino acid sequence

   $(Glu)_m$-$(Asp)_n$-Pro

   in which m is zero or 1, and n is 1, 2 or 3, and is proteolytically cleaved.

5. Bacterial expression vector containing at least one gene coding for CSF as claimed in claim 1.

6. A bacterial cell containing a vector as claimed in claim 5.

7. E. coli containing a vector as claimed in claim 5.

8. CSF for use in medical treatment.

9. The use of CSF as claimed in claim 1 for the preparation of medicaments.

**Revendications**
**Revendications pour les Etats contractants suivants : BE, CH, DE, FR, GB, IT, LI, LU, NL, SE**

1. Protéines de type facteur "stimulant la formation de colonies" de granulocytes-macrophages humain (GM-CSF) de formule

Pro-$(As)_x$-CSF(12-126)-Z,

dans laquelle $(AS)_x$ représente en partie ou en totalité les 11 premiers aminoacides de la Séquence GM-CSF native et Z représente Glu ou Asp, dans ce qui suit "CSF".

2. Procédé pour la production de CSF selon la revendication 1, caractérisé en ce que l'on incorpore un gène codant pour le CSF dans un vecteur d'expression bactérien, on transforme avec celui-ci des bactéries, en particulier E. coli, et on l'exprime dans celles-ci.

3. Procédé selon la revendication 2, caractérisé en ce que l'on exprime le CSF sous forme d'une protéine de fusion qui est ensuite coupée chimiquement par voie enzymatique.

4. Procédé selon la revendication 3, caractérisé en ce que la protéine de fusion contient à l'extrémité N-terminale, au voisinage du CSF, la séquence d'aminoacides

$(Glu)_m(Asp)_n$-Pro,

dans laquelle m est zéro ou 1 et n est 1, 2 ou 3, et est coupée par protéolyse.

5. Vecteur d'expression bactérien, contenant au moins un gène codant pour le CSF, selon la revendication 1.

6. Cellules bactériennes, en particulier de E. coli, contenant un vecteur selon la revendication 5.

7. Médicament, contenant du CSF ou constitué de CSF selon la revendication 1.

8. CSF selon la revendication 1, pour utilisation en thérapeutique.

9. Utilisation du CSF selon la revendication 1, pour la fabrication de médicaments.

**Revendications pour les Etats contractants suivants : ES, AT**

1. Procédé pour la production de protéines de type facteur "stimulant la formation de colonies" de granulocytes-macrophages humain (GM-CSF) de formule

Pro-$(As)_x$-CSF(12-126)-Z,

dans laquelle $(AS)_x$ représente en partie ou en totalité les 11 premiers aminoacides de la séquence GM-CSF native et Z représente Glu ou Asp, caractérisé en ce que l'on incorpore dans un vecteur d'expression bactérien un gène codant pour le CSF, on transforme avec celui-ci des bactéries et on l'exprime dans celles-ci.

2. Procédé selon la revendication 1, caractérisé en ce que la bactérie est E. coli.

3. Procédé selon la revendication 1 ou 2, caractérisé en ce que l'on exprime le CSF sous forme d'une protéine de fusion qui est ensuite coupée chimiquement ou par voie enzymatique.

4. Procédé selon la revendication 3, caractérisé en ce que la protéine de fusion contient à l'extrémité N-terminale, voisine du CSF, la séquence d'aminoacides

$(Glu)_m$-$(Asp)_n$-Pro,

dans laquelle m est zéro ou 1 et n est 1, 2 ou 3, et est coupée par protéolyse.

**Revendications pour l'Etat contractant suivant : GR**

1. Procédé pour la production de protéines de type facteur "stimulant la formation de colonies" de granulocytes-macrophages humain (GM-CSF) de formule

Pro-$(As)_x$-CSF(12-126)-Z,

dans laquelle $(AS)_x$ représente en partie ou en totalité les 11 premiers aminoacides de la séquence GM-CSF native et Z représente Glu ou Asp, caractérisé en ce que l'on incorpore dans un vecteur d'expression bactérien un gène codant pour le CSF, on transforme avec celui-ci des bactéries et on l'exprime dans celles-ci.

2. Procédé selon la revendication 1, caractérisé en ce que la bactérie est E. coli.

3. Procédé selon la revendication 1 ou 2, caractérisé en ce que l'on exprime le CSF sous forme d'une protéine de fusion qui est ensuite coupée chimiquement ou par voie enzymatique.

4. Procédé selon la revendication 3, caractérisé en ce que la protéine de fusion contient à l'extrémité N-terminale, voisine du CSF, la séquence d'aminoacides

$(Glu)_m$-$(Asp)_n$-Pro,

dans laquelle m est zéro ou 1 et n est 1, 2 ou 3, et est coupée par protéolyse.

5. Vecteur d'expression bactérien, contenant au moins un gène codant pour le CSF selon la revendication 1.

6. Cellules bactériennes, contenant un vecteur selon la revendication 5.

7. E. coli contenant un vecteur selon la revendication 5.

8. CSF selon la revendication 1 pour utilisation en thérapeutique.

9. Utilisation du CSF selon la revendication 1 pour la fabrication de médicaments.

FIG. 1

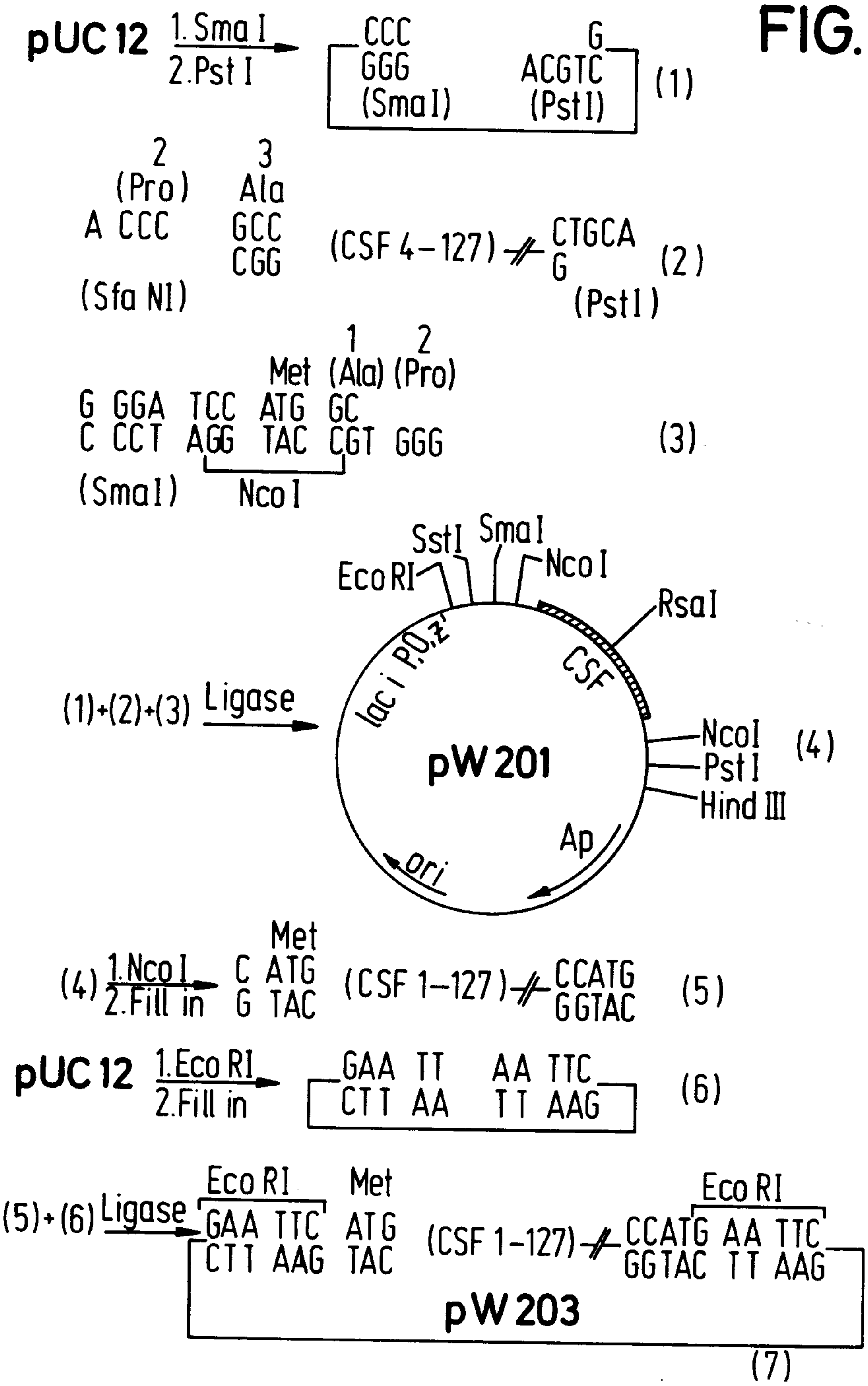
pUC 12
1. Sma I
2. Pst I
CCC
GGG
(Sma I)
G
ACGTC
(Pst I)
(1)
2
(Pro)
3
Ala
A CCC
GCC
CGG
(CSF 4–127)
CTGCA
G
(2)
(Sfa NI)
(Pst I)
1
2
Met
(Ala)
(Pro)
G GGA TCC ATG GC
C CCT AGG TAC CGT GGG
(3)
(Sma I)
Nco I
Sst I
Sma I
Eco RI
Nco I
Rsa I
lac i P,O,z'
CSF
(1)+(2)+(3)
Ligase
pW 201
Nco I
Pst I
Hind III
(4)
Ap
ori
Met
(4)
1. Nco I
2. Fill in
C ATG
G TAC
(CSF 1–127)
CCATG
GGTAC
(5)
pUC 12
1. Eco RI
2. Fill in
GAA TT
CTT AA
AA TTC
TT AAG
(6)
Eco RI
Met
Eco RI
(5)+(6)
Ligase
GAA TTC
CTT AAG
ATG
TAC
(CSF 1–127)
CCATG AA TTC
GGTAC TT AAG
pW 203
(7)

FIG. 1a

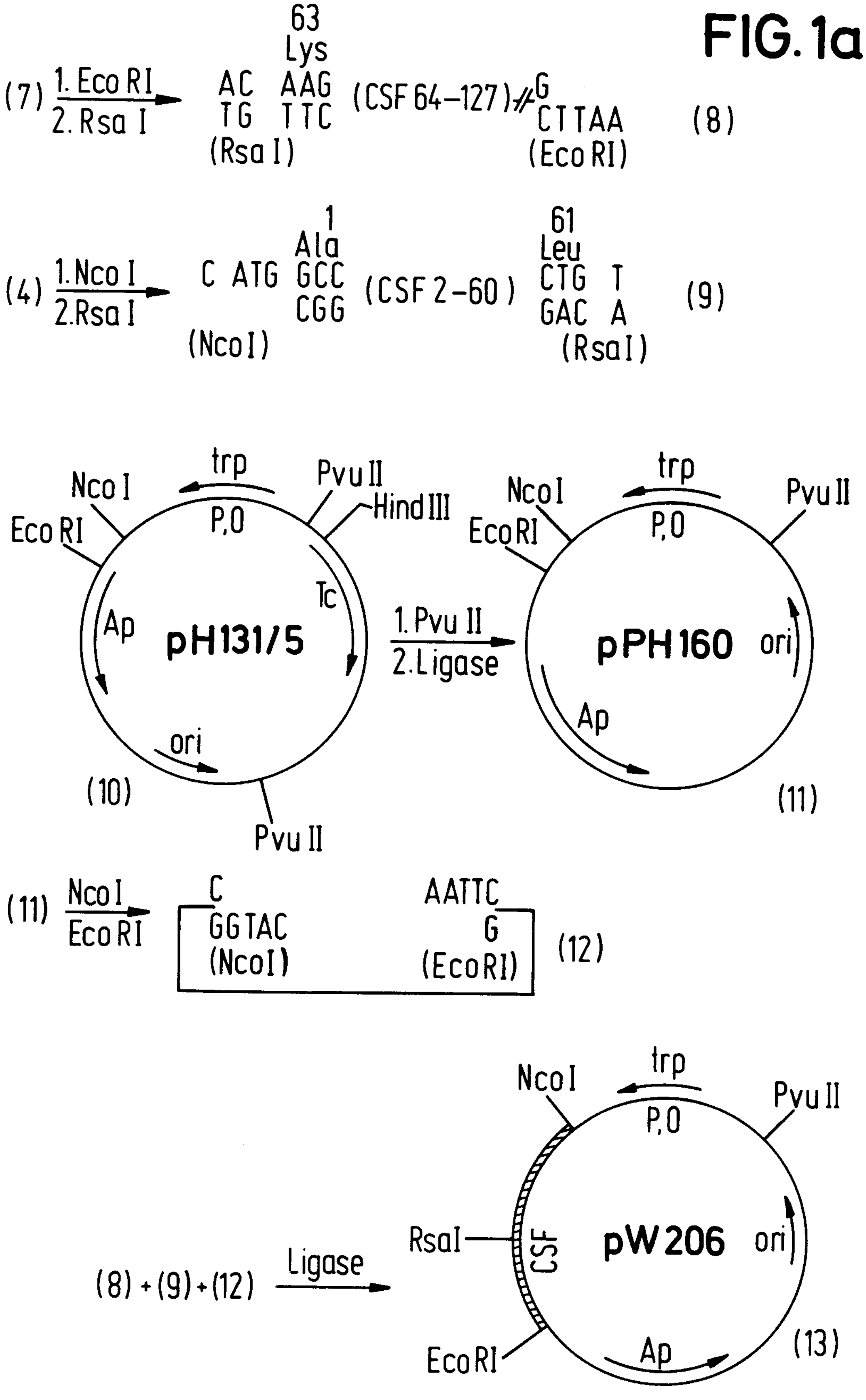
(7)
1. Eco RI
2. Rsa I
63
Lys
AC AAG
TG TTC
(Rsa I)
(CSF 64–127)
G
CTTAA
(Eco RI)
(8)
(4)
1. Nco I
2. Rsa I
1
Ala
C ATG GCC
CGG
(Nco I)
(CSF 2–60)
61
Leu
CTG T
GAC A
(Rsa I)
(9)
trp
Nco I
Eco RI
P,O
Pvu II
Hind III
Tc
Ap
pH131/5
ori
(10)
Pvu II
1. Pvu II
2. Ligase
trp
Nco I
Eco RI
P,O
Pvu II
pPH160
ori
Ap
(11)
(11)
Nco I
Eco RI
C
GGTAC
(Nco I)
AATTC
G
(Eco RI)
(12)
(8) + (9) + (12)
Ligase
trp
Nco I
P,O
Pvu II
Rsa I
CSF
pW206
ori
Eco RI
Ap
(13)

FIG. 1b

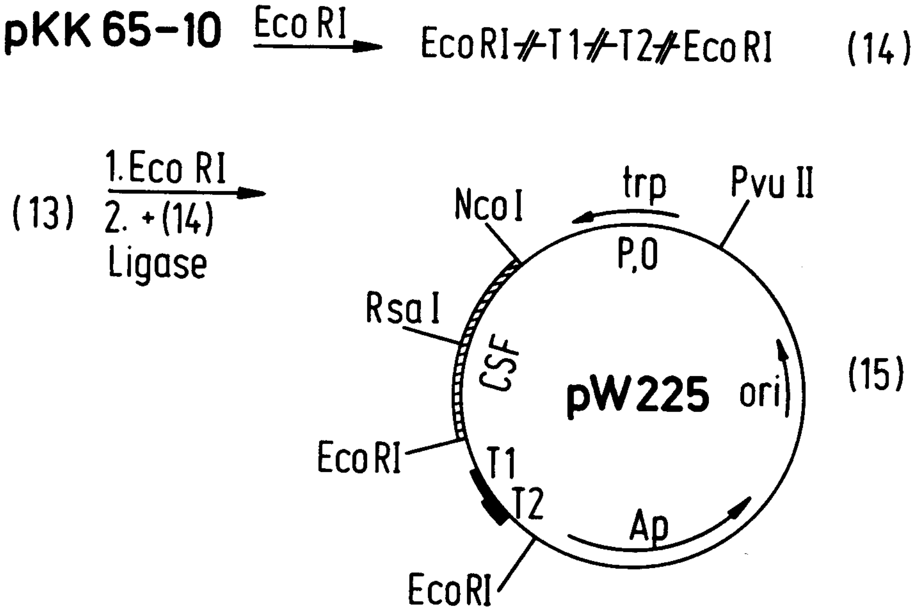
pKK 65-10
EcoRI
EcoRI≠T1≠T2≠EcoRI
(14)
(13)
1. Eco RI
2. + (14)
Ligase
NcoI
trp
PvuII
P,O
RsaI
CSF
pW225
ori
(15)
EcoRI
T1
T2
Ap
EcoRI

FIG. 2

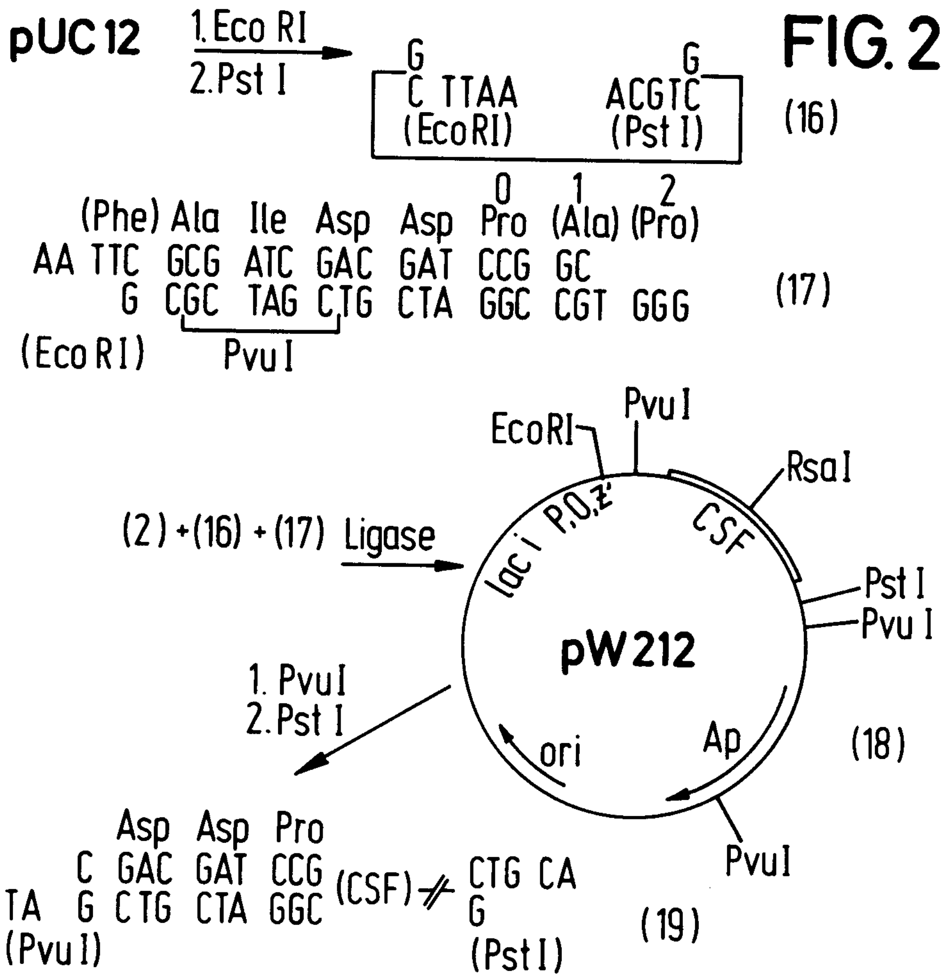
pUC12
1. Eco RI
2. Pst I
G
C TTAA
(EcoRI)
G
ACGTC
(Pst I)
(16)
0 1 2
(Phe) Ala Ile Asp Asp Pro (Ala) (Pro)
AA TTC GCG ATC GAC GAT CCG GC
G CGC TAG CTG CTA GGC CGT GGG
(17)
(EcoRI)
PvuI
EcoRI
PvuI
RsaI
(2) + (16) + (17) Ligase
P,O,z'
lac i
CSF
PstI
PvuI
pW212
1. PvuI
2. Pst I
ori
Ap
(18)
Asp Asp Pro
C GAC GAT CCG
TA G CTG CTA GGC
(PvuI)
(CSF)≠
CTG CA
G
(PstI)
(19)
PvuI

FIG. 2a

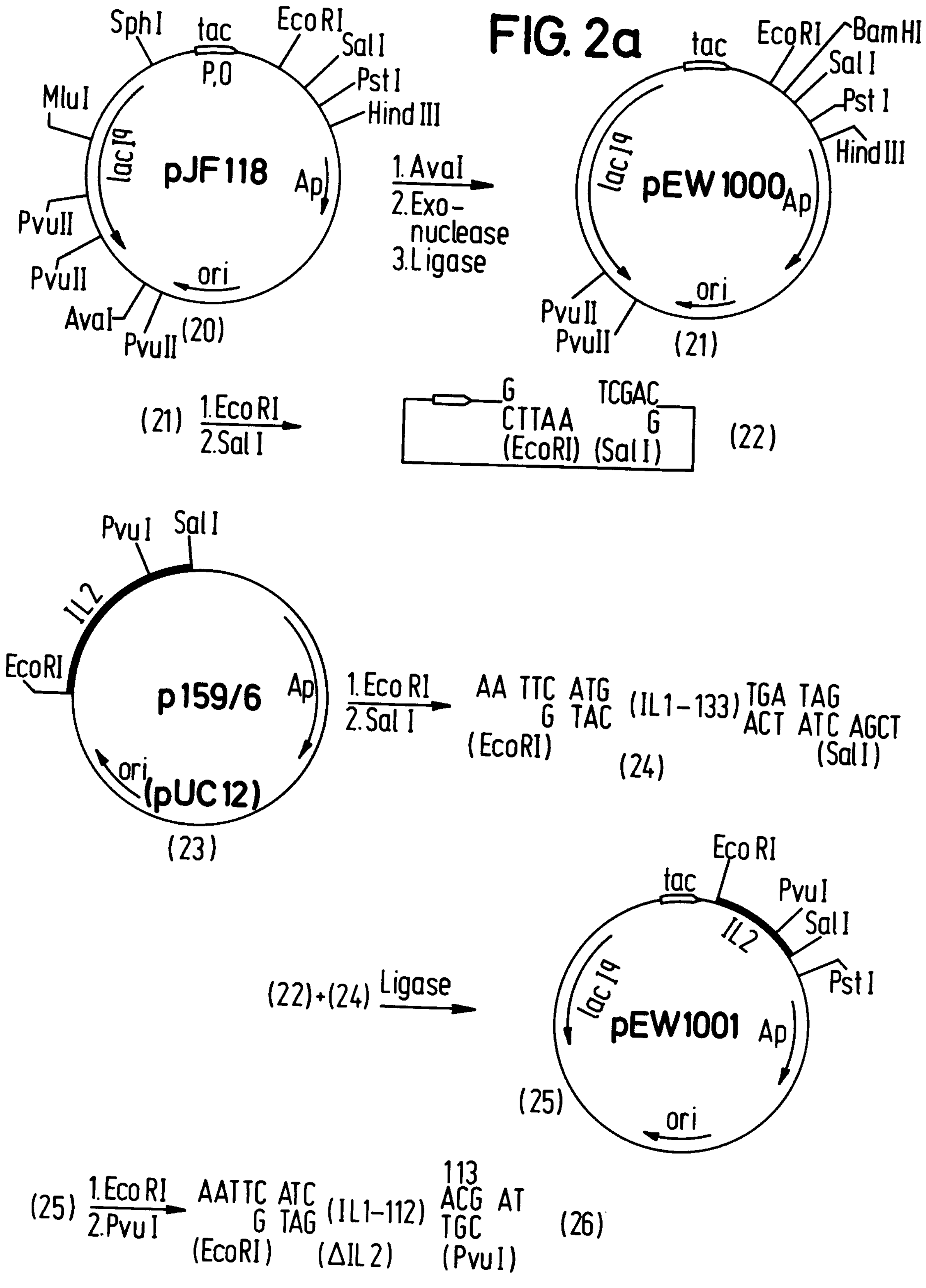

SphI
tac
P,O
EcoRI
SalI
PstI
HindIII
MluI
lacIq
pJF118
Ap
PvuII
PvuII
ori
AvaI
PvuII
(20)
1. AvaI
2. Exo-nuclease
3. Ligase
tac
EcoRI
BamHI
SalI
PstI
HindIII
lacIq
pEW1000
Ap
ori
PvuII
PvuII
(21)
(21)
1. EcoRI
2. SalI
G
CTTAA
(EcoRI)
TCGAC
G
(SalI)
(22)
PvuI
SalI
IL2
EcoRI
p159/6
Ap
ori
(pUC12)
(23)
1. EcoRI
2. SalI
AA TTC ATG
G TAC
(EcoRI)
(IL1-133)
TGA TAG
ACT ATC AGCT
(SalI)
(24)
EcoRI
tac
PvuI
SalI
IL2
PstI
(22)+(24)
Ligase
lacIq
pEW1001
Ap
ori
(25)
(25)
1. EcoRI
2. PvuI
AATTC ATC
G TAG
(EcoRI)
(IL1-112)
(ΔIL2)
113
ACG AT
TGC
(PvuI)
(26)

FIG. 2b

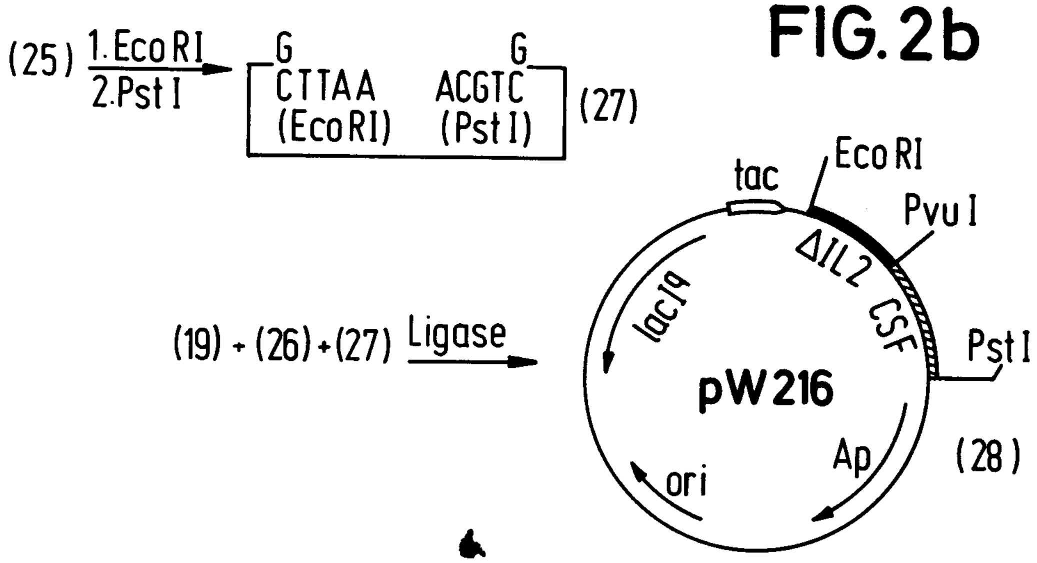
(25) 1. EcoRI 2. PstI
G G
CTTAA ACGTC
(EcoRI) (PstI)
(27)
(19) + (26) + (27) Ligase
tac
EcoRI
PvuI
ΔIL2
CSF
PstI
lacIq
pW216
Ap
ori
(28)

FIG. 3

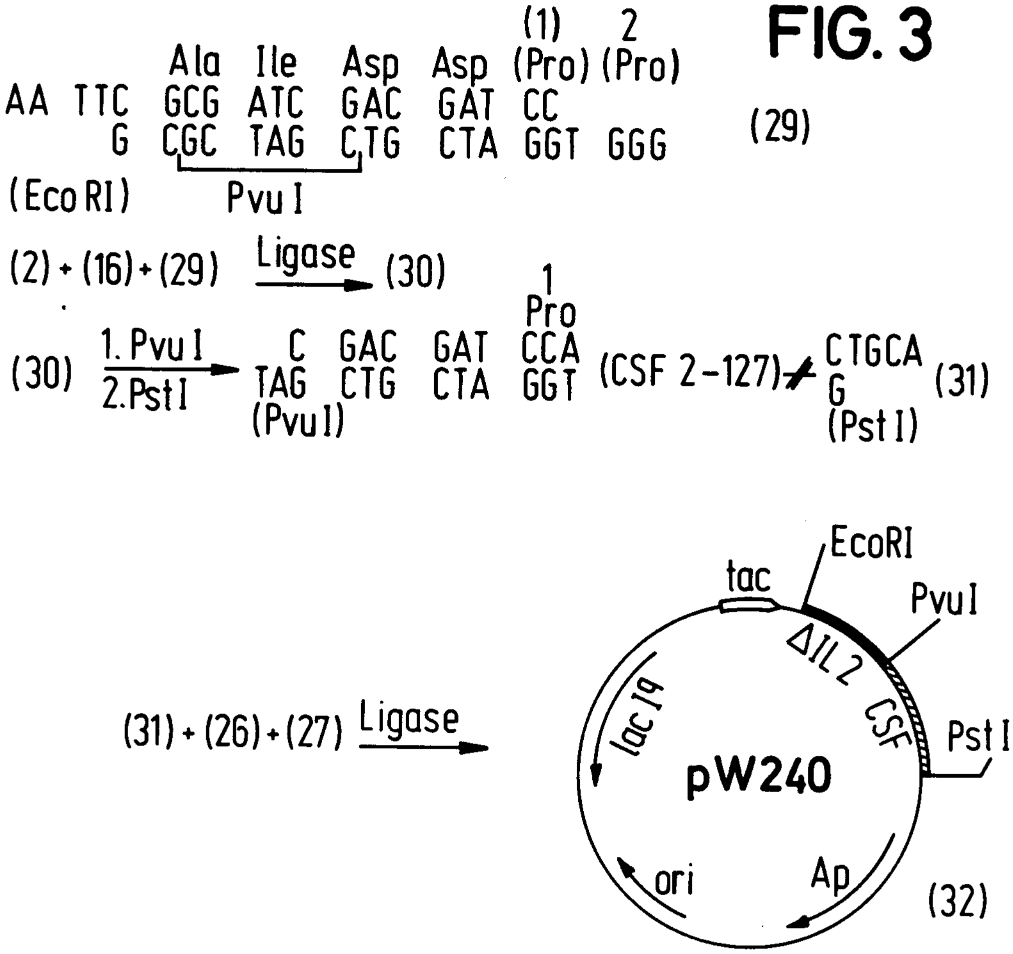
(1) 2
Ala Ile Asp Asp (Pro) (Pro)
AA TTC GCG ATC GAC GAT CC
G CGC TAG CTG CTA GGT GGG
(29)
(EcoRI) PvuI
(2) + (16) + (29) Ligase (30)
1
Pro
(30) 1. PvuI 2. PstI
C GAC GAT CCA (CSF 2-127) CTGCA
TAG CTG CTA GGT G
(PvuI) (PstI)
(31)
(31) + (26) + (27) Ligase
tac
EcoRI
PvuI
ΔIL2
CSF
PstI
lacIq
pW240
ori
Ap
(32)

FIG. 4

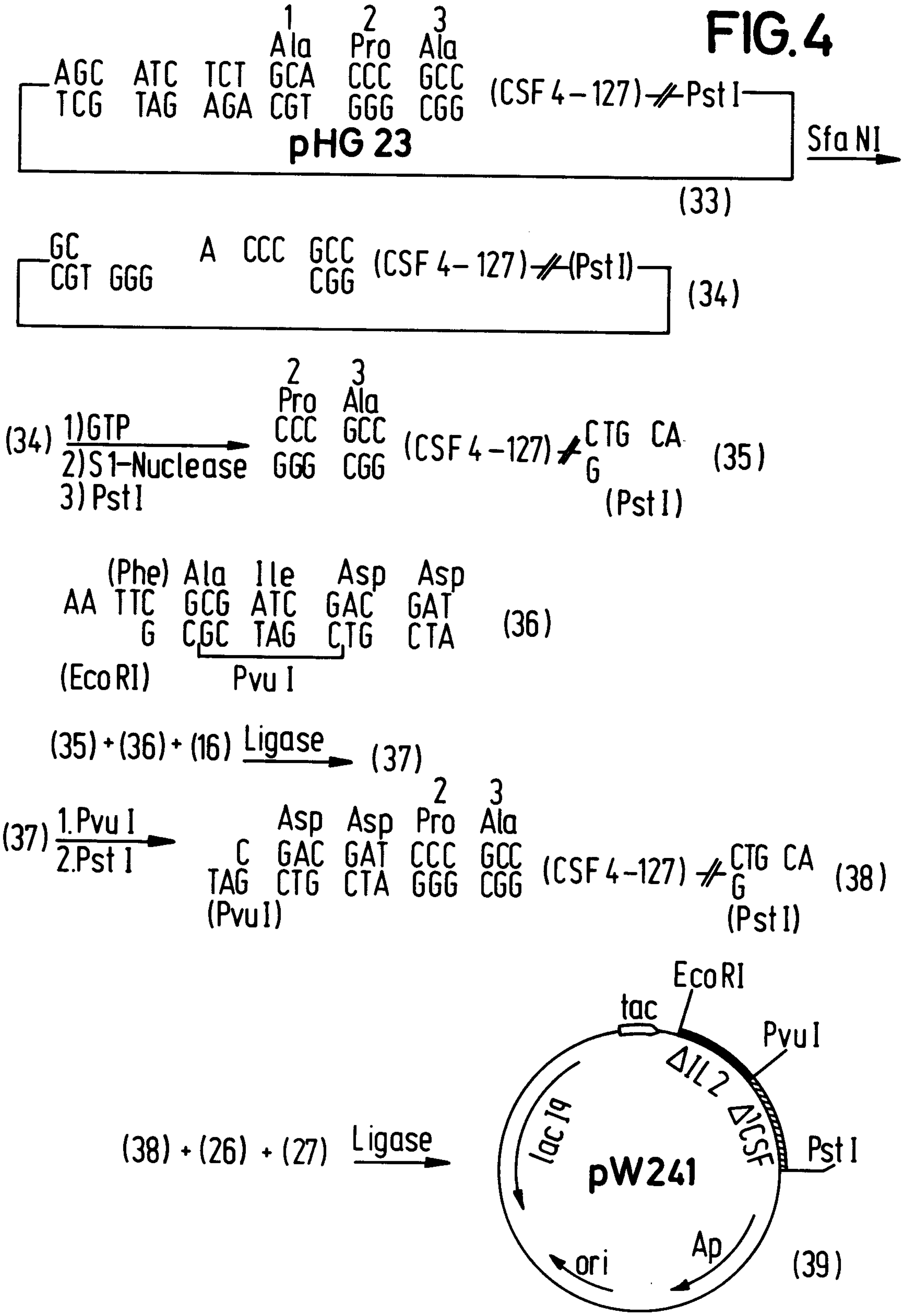
1 2 3
Ala Pro Ala
AGC ATC TCT GCA CCC GCC
TCG TAG AGA CGT GGG CGG
(CSF 4−127) Pst I
pHG 23
Sfa NI
(33)
GC A CCC GCC
CGT GGG CGG
(CSF 4− 127) (Pst I)
(34)
(34) 1) GTP 2) S1-Nuclease 3) Pst I
2 3
Pro Ala
CCC GCC
GGG CGG
(CSF 4−127) CTG CA G
(Pst I)
(35)
(Phe) Ala Ile Asp Asp
AA TTC GCG ATC GAC GAT
G CGC TAG CTG CTA
(Eco RI) Pvu I
(36)
(35) + (36) + (16) Ligase (37)
(37) 1. Pvu I 2. Pst I
Asp Asp Pro Ala
2 3
C GAC GAT CCC GCC
TAG CTG CTA GGG CGG
(Pvu I)
(CSF 4−127) CTG CA G
(Pst I)
(38)
(38) + (26) + (27) Ligase
Eco RI
tac
Pvu I
ΔIL 2
Δ'CSF
Pst I
lac Iq
pW241
Ap
ori
(39)

**FIG. 5**

```
                         13
                        (Trp)
(33)  1.Pst I          TGG  (CSF 14–127) ⫽ CTG  CA      (40)
      2.Bst NI,part.   CC                  G
                       (Bst NI)               (Pst I)
```

```
 6     7     8     9    10    11    12
Pro   Ser   Pro   Ser   Thr   Gln   Pro
CCG   TCT   CCG   TCT   ACG   CAG   CCC       (41)
GGC   AGA   GGC   AGA   TGC   GTC   GGG  A
```

(41) + (36) —Ligase→ (42)

(42) + (40) + (16) —Ligase→

```
                               Asp   Asp
 GAA   TTC    GCG   ATC    GAC   GAT   (CSF 6–127) ⫽ CTG   CAG
 CTT   AAG    CGC   TAG    CTG   CTA                 GAC   GTC      (43)
   EcoRI        Pvu I                pW 212             Pst I
```

(43) —1. Pvu I / 2. Pst I→ (44)

(44) + (26) + (27) —Ligase→

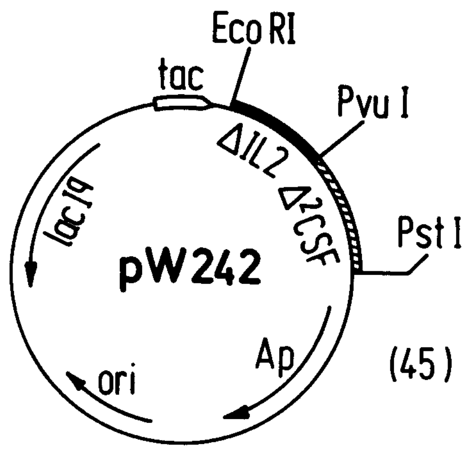


(45)

**FIG. 6**

```
 8     9    10    11    12
Pro   Ser   Thr   Gln   Pro
CCG   TCT   ACG   CAG   CCC      (46)
GGC   AGA   TGC   GTC   GGG A
```

(46) + (36) —Ligase→ (47)

(47) + (40) + (16) —Ligase→ (48)

(48) —1. Pvu I / 2. Pst I→ (49)

(49) + (26) + (27) ——→ **pW 243** (50)

FIG. 7

```
                     12
        Asp  Asp    Pro
AA TTC GCG ATC GAC  GAT  CCC     (51)
     G CGC TAG CTG  CTA  GGG A
(EcoRI)  (PvuI)         (BstNI)
```

(51) + (33) + (16) --Ligase--> (52)

(52) --1. PvuI / 2. PstI--> (53)

(53) + (26) + (27) --Ligase--> **pW 244** (54)

FIG. 8

```
                                              125
                 Asp  Asp  Pro                Val
(19) --BstNI-->  C  GAC  GAT  CCG              GTC  C
      part.     TAG CTG  CTA  GGC (CSF1-124)  CAG  GT   (55)
               (PvuI)                            (BstNI)

                  (Ser) Ile  Ser                125
(33) --1. PstI-->    GC ATC  TCT               GTC  C
   2. BstNI,     ACG TCG TAG AGA  (CSF1-124)   CAG  GT    (56)
      part.      (PstI)                        (BstNI)

   Asp  Stp  Stp            PstI
AG GAT  TGA  TAA  GCT TTC  TGC  AGC  CC   (57)
 C CTA  ACT  ATT  CGA AAG  ACG  TCG  GG
(BstNI)                             (SmaI)
```

```
pUC 13 --1. PstI / 2. SmaI-->  CTGCA      GGG      (58)
                               G          CCC
                               (PstI)     (SmaI)
```

(58) + (56) + (57) --Ligase-->

```
                                125   126
PstI  Ser  Ile  Ser             Val   Gln  Asp  Stp  Stp          PstI       SmaI
C TGC AGC  ATC  TCT            GTC   CAG  GAT  TGA  TAA  GCT TTC TGC AGC CCGGG
G ACG TCG  TAG  AGA (CSF1-124) CAG   GTC  CTA  ACT  ATT  CGA AAG ACG TCG GGCCC
                          pW 245                   HindIII
                                                                        (59)
```

FIG.8a

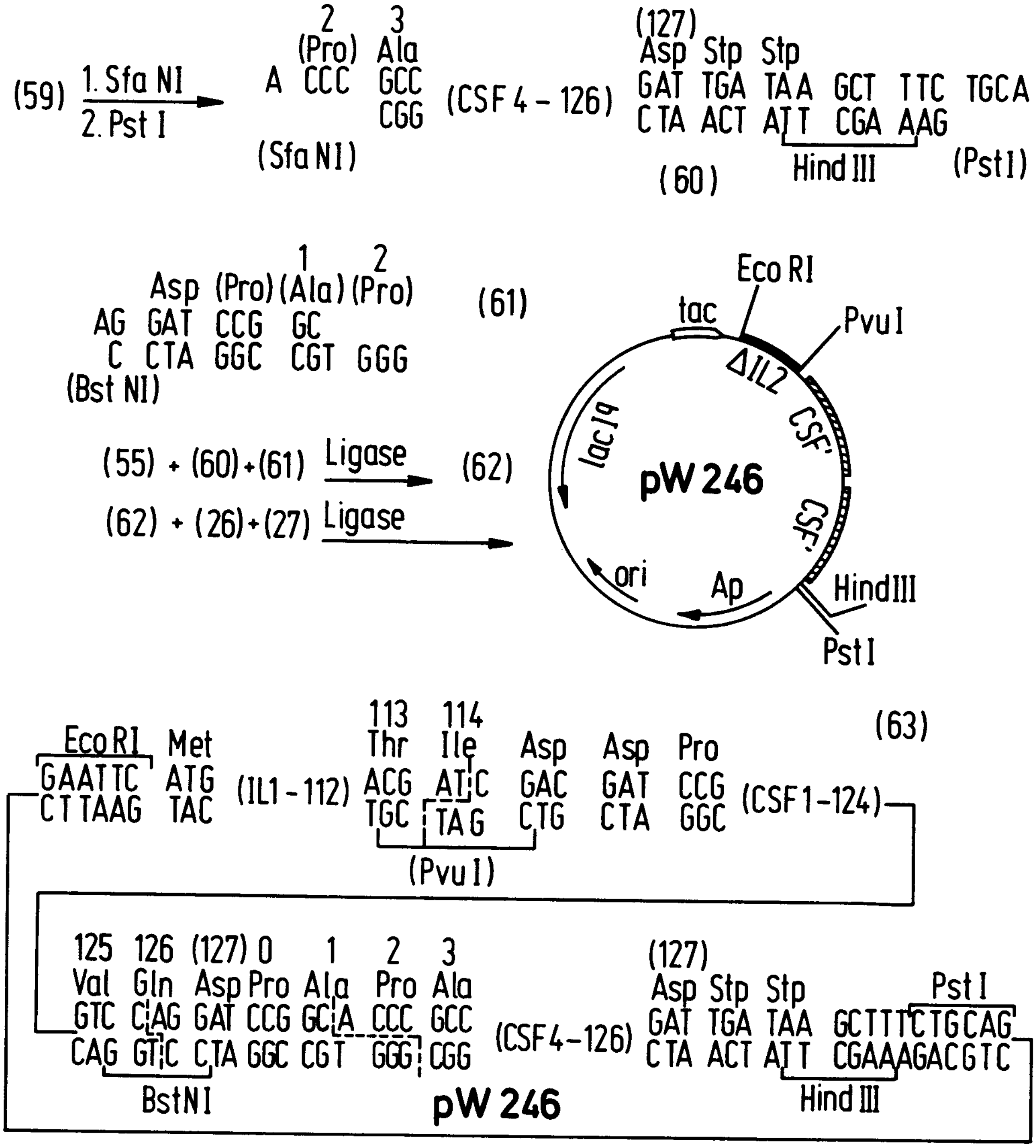
(59)
1. Sfa NI
2. Pst I
2 3
(Pro) Ala
A CCC GCC
CGG
(SfaNI)
(CSF 4 - 126)
(127)
Asp Stp Stp
GAT TGA TAA GCT TTC TGCA
CTA ACT ATT CGA AAG
(60)
Hind III
(PstI)
1 2
Asp (Pro) (Ala) (Pro)
AG GAT CCG GC
C CTA GGC CGT GGG
(Bst NI)
(61)
(55) + (60) + (61) Ligase (62)
(62) + (26) + (27) Ligase
Eco RI
tac
PvuI
ΔIL2
CSF'
lacIq
pW 246
CSF'
HindIII
ori
Ap
PstI
(63)
EcoRI Met
GAATTC ATG
CTTAAG TAC
(IL1 - 112)
113 114
Thr Ile Asp Asp Pro
ACG ATC GAC GAT CCG
TGC TAG CTG CTA GGC
(PvuI)
(CSF1-124)
125 126 (127) 0 1 2 3
Val Gln Asp Pro Ala Pro Ala
GTC CAG GAT CCG GCA CCC GCC
CAG GTC CTA GGC CGT GGG CGG
BstNI
(CSF4-126)
(127)
Asp Stp Stp
GAT TGA TAA GCTTTCTGCAG
CTA ACT ATT CGAAAGACGTC
PstI
Hind III
pW 246

# FIG. 9

(63) —1. Eco RI / 2. Pst I→ (EcoRI)–ΔIL2–CSF'–CSF'–(Pst I) (64)

(64) —Rsa I / part.→ (Eco RI)–ΔIL2–CSF'–(CSF'1–60) 61 Leu CTG T / GAC A (RsaI) (65)

+ AC / CG (RsaI) 63 Lys AAG / TTC (CSF 64–126) (127) Asp GAT TGA TAA GCT TTC TGAC / CTA ACT ATT CGA AAG (Pst I) (66)

(66) —Bst NI→ AC / TG (RsaI) 63 Lys AAG / TTC (CSF 64–124) 125 Val GTC C / CAG GT (BstNI) (67)

(27) + (65) + (67) + (61) + (60) —Ligase→

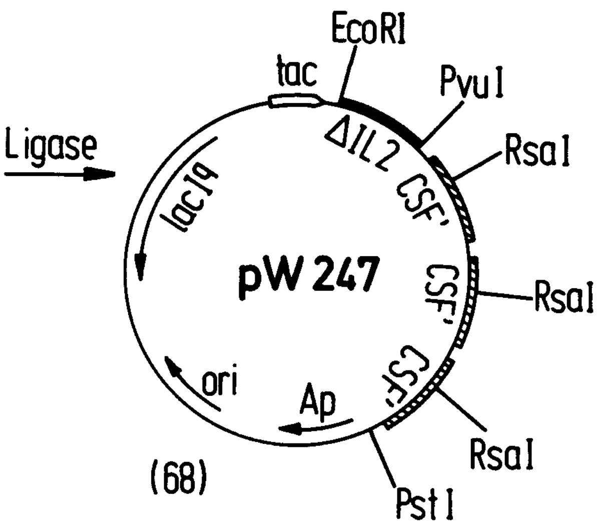

FIG.10

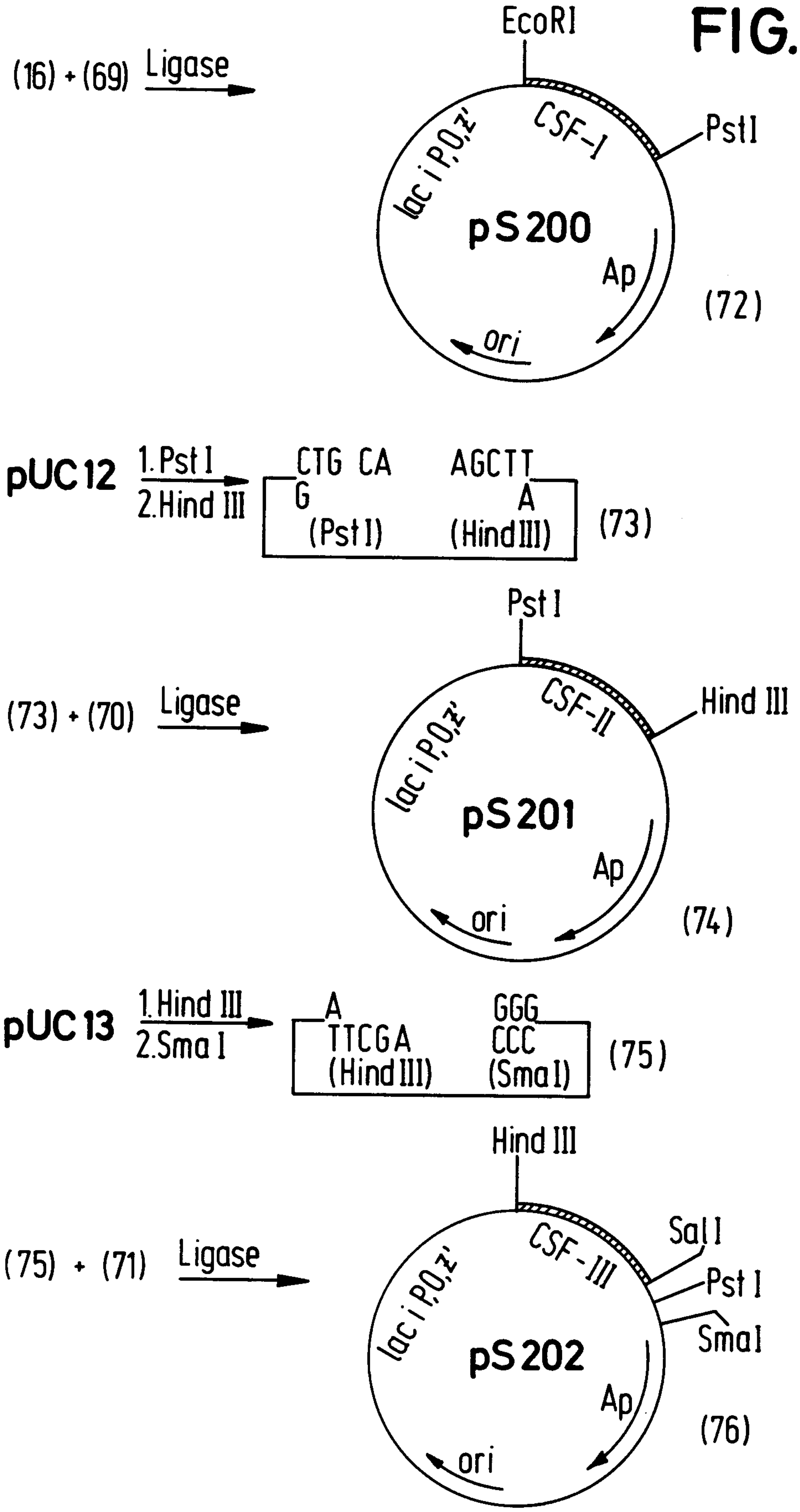
(16) + (69) Ligase
EcoRI
PstI
CSF-I
lac i P,O,z'
pS200
Ap
ori
(72)
pUC12
1. Pst I
2. Hind III
CTG CA
G
(Pst I)
AGCTT
A
(Hind III)
(73)
(73) + (70) Ligase
Pst I
Hind III
CSF-II
lac i P,O,z'
pS201
Ap
ori
(74)
pUC13
1. Hind III
2. Sma I
A
TTCGA
(Hind III)
GGG
CCC
(Sma I)
(75)
(75) + (71) Ligase
Hind III
Sal I
Pst I
Sma I
CSF-III
lac i P,O,z'
pS202
Ap
ori
(76)

FIG.10a

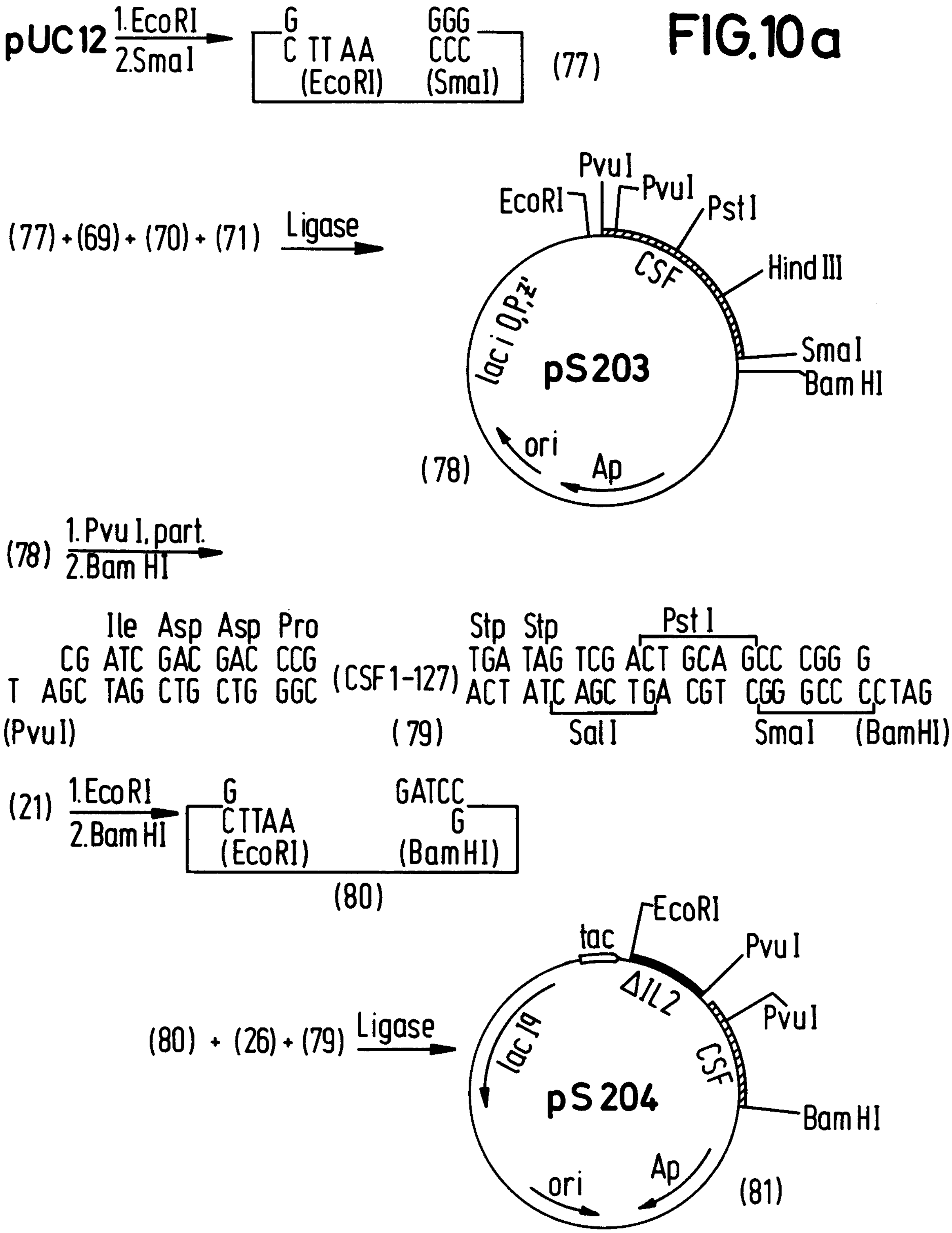
pUC12
1. EcoRI
2. SmaI
G
C TT AA
(EcoRI)
GGG
CCC
(SmaI)
(77)
(77) + (69) + (70) + (71)
Ligase
EcoRI
PvuI
PvuI
PstI
HindIII
SmaI
BamHI
CSF
laci O,P,z'
pS203
ori
Ap
(78)
(78)
1. PvuI, part.
2. BamHI
Ile Asp Asp Pro
CG ATC GAC GAC CCG
T AGC TAG CTG CTG GGC
(PvuI)
(CSF1-127)
(79)
Stp Stp
PstI
TGA TAG TCG ACT GCA GCC CGG G
ACT ATC AGC TGA CGT CGG GCC CCTAG
SalI
SmaI
(BamHI)
(21)
1. EcoRI
2. BamHI
G
CTTAA
(EcoRI)
GATCC
G
(BamHI)
(80)
(80) + (26) + (79)
Ligase
tac
EcoRI
PvuI
PvuI
ΔIL2
CSF
lacIq
pS204
BamHI
ori
Ap
(81)

FIG.11

PvuI
1 2 3 4 5 6 7 8
(Ser) Ile Ala Trp Ala Pro Ala Arg Ser Pro (Ser) (Pro)
AAT TCG ATC GCA TGG GCG CCG GCC CGA TCG CCG (82)
GC TAG CGT ACC CGC GGC CGG GCT AGC GGC AGA GGC
(EcoRI)

7 8 9 10 11 12 (13) 14 15 16
(Ser) (Pro) Ser Thr Gln Pro His Glu His Val
TCT CCG TCG ACC CAG CCC CAC GAA CAC GTT (83)
AGC TGG GTC GGG GTG CTT GTG CAA
(HpaI)

17
Asn
(72) 1) EcoRI / 2) HpaI → G / CTTAA (EcoRI) AAC / TTG (HpaI) (CSF 18–50) (84)

(84) + (82) + (83) Ligase →

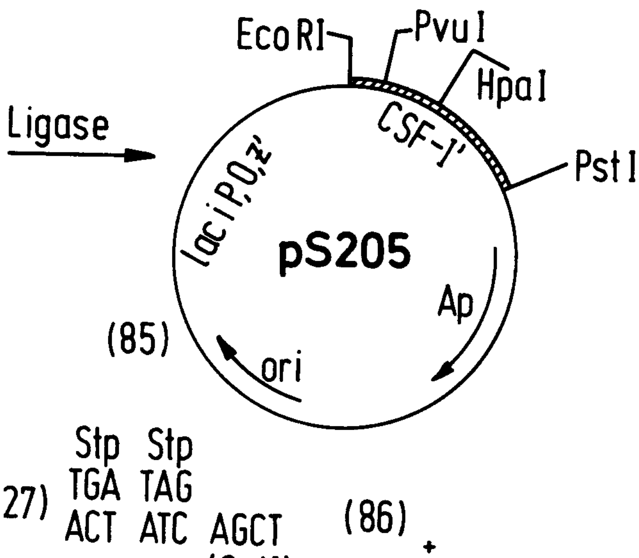


(85)

73
(Leu)
(76) 1.HindIII / 2.SalI → AG CTT / A (HindIII) (CSF 74 -127) Stp Stp / TGA TAG / ACT ATC AGCT (SalI) (86) +

PstI SmaI
+ A / TTCGA (HindIII) TCG ACT GCA GCC CGG G / TA CGT CGG GCC C (SalI) (87)

(Leu) (Phe)
(86) TaqI → AGCTT / A (HindIII) (CSF 74 -118) TT / AAG C (TaqI) (88)

120 121 (122) 123 124 125 126 127
Asp Cys His Glu Pro Val Gln Glu Stp Stp
C GAC TGT CAC GAG CCG GTC CAG GAA TGA TAG (89)
TG ACA GTG CTC GGC CAG GTC CTT ACT ATC AGC T
(TaqI) (SalI)

FIG.11a

(87) + (88) + (89) Ligase →

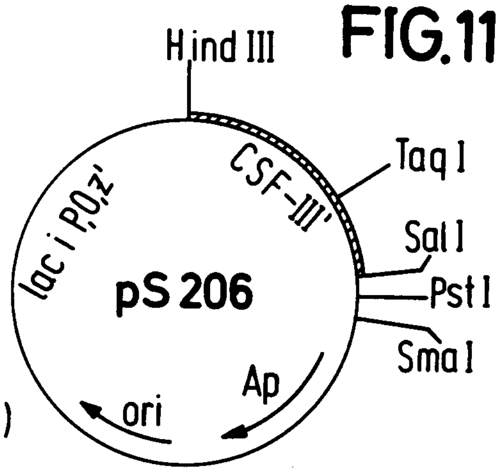


(90)

(90) 1. Hind III / 2. Sal I → (91)

(85) 1. Pvu I, part. / 2. Pst I → (92)

(22)+(26)+(92)+(70)+(91) Ligase →

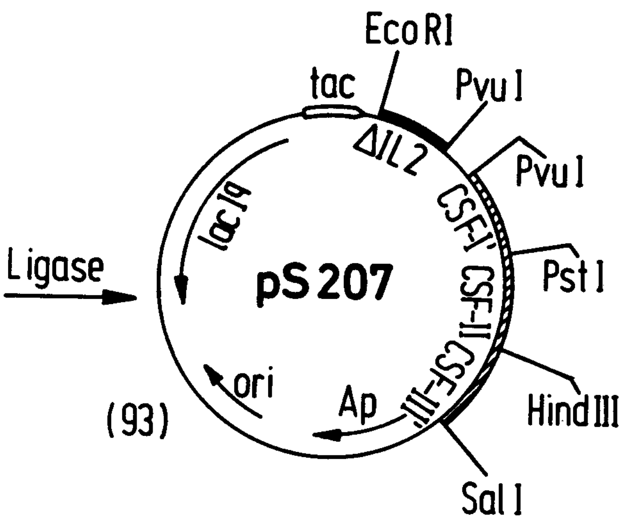


(93)

FIG.12

| | | | | | | | | | |
|---|---|---|---|---|---|---|---|---|---|
| 95 | 96 | 97 | 98 | 90 | (100) | 101 | 102 | 103 | |
| (Ser) | (Cys) | Ala | Thr | Gln | Thr | Ile | (Thr) | (Phe) | |
| TCT | TGC | GCG | ACG | CAG | ACC | ATC | | | (94) |
| | | CGT | TGC | GTC | TGG | TAG | TGG | AAG | |

FIG.13

33 34 35 (36) 37 38 39 40 41 42 43
(Ala) Ala Glu Ile Asn Glu Thr Val Glu (Val) (Ile)
G GCC GCG GAA ATC AAC GAA ACC GTT GAA
CGG CTT TAG TTG CTT TGG CAA CTT CAC TAT
(XmaIII)
(95)

42 43 44 45 (46) 47 48 49
(Val) (Ile) Ser Glu Leu Phe Asp (Leu)
GTG ATA TCT GAG CTC TTC GAC CTG CA
AGA CTC GAG AAG CTG G
(PstI)
(96)

(72) 1.PvuI / 2.XmaIII →

32 33
(Thr) (Ala)
CG AC
T AGC (CSF6-31) TGC CGG
(PvuI) (XmaIII)
(97)

1 2 3 4
Ile Ala Met Ala Pro Ala (Arg)
AAT TCG ATC GCG ATG GCG CCG GCC CGA TC
GC TAG CGC TAC CGC GGC CGG G
(EcoRI) (PvuI)
(98)

(16) + (98) + (97) + (95) + (96) → **pS208** (99)

73 74 75 76 77 78 (79) (80) 81
(Leu) Lys Gly Pro Leu Thr Leu Leu (Ala)
AG CTT AAG GGG CCC CTC ACC CTG CTG G
A TTC CCC GGG GAG TGG GAC GAC CGA TC
(HindIII) (NheI)
(100)

(76) 1.HindIII / 2.NheI →
A CTAGC
TTCGA G
(HindIII) (NheI)
(101)

(100) + (101) Ligase → **pS209** (102)

FIG.13a

```
(93)  1. Pvu I, part.  →   ┌(IL-2 1–112) ACGAT      TCGAC┐
      2. Sal I             │             TGC            G │  (103)
                           │             (PvuI)    (SalI) │
                           └──────────────────────────────┘

(99)  1. Pvu I, part.  →
      2. Pst I

                                                     (46) 47  48  49
    Ala Met                   Ile                    Leu Phe Asp (Leu)
  C GCG ATG  (CSF 1-35)  ATC  (CSF 37-45)  CTG TTC GAC CTG CA
TAG CGC TAC              TAG               GAG AAG CTG G
(Pvu I)                         (104)                   (Pst I)

(102)  1. Hind III  →
       2. Sal I

   73                  (79) (80)
   Leu                 Leu  Leu
AG CCT  (CSF 74-78)    CTG  CTG  (CSF 81-127)  TGA TGA
    A                  GAC  GAC                ACT ACT AGCT
(Hind III)                       (105)                 (SalI)

(103) + (104) + (70) + (105)  Ligase →  pS210  (106)
```

FIG.14

```
   73    74  75  76  77  78      81
  (Leu) Lys Gly Pro Leu Thr Leu (Ala)
AG CTT  AAG GGG CCC CTC ACC CTG G          (107)
     A  TTC CCC GGG GAG TGG GAC CGA TC
(Hind III)                      (NheI)
```

FIG.15

```
   73   74  75  76  77  78  81
  Leu  Lys Gly Pro Leu Thr (Ala)
AG CTT AAG GGG CCC CTC ACC G           (108)
     A TTC CCC GGG GAG TGG CGA TC
(Hind III)                 (NheI)
```